(19) Europäisches Patentamt
European Patent Office
Office européen des brevets

(11) **EP 1 663 337 B1**

(12) **EUROPÄISCHE PATENTSCHRIFT**

(45) Veröffentlichungstag und Bekanntmachung des Hinweises auf die Patenterteilung:
**21.03.2012 Patentblatt 2012/12**

(21) Anmeldenummer: **04765404.1**

(22) Anmeldetag: **18.09.2004**

(51) Int Cl.:
*A61L 31/04* (2006.01)   *A61L 31/10* (2006.01)
*A61L 27/14* (2006.01)   *A61L 27/34* (2006.01)
*A61L 27/54* (2006.01)   *A61L 33/00* (2006.01)

(86) Internationale Anmeldenummer:
**PCT/EP2004/010514**

(87) Internationale Veröffentlichungsnummer:
**WO 2005/030287 (07.04.2005 Gazette 2005/14)**

(54) **WIRKSTOFFABGEBENDE GEFÄSSPROTHESE**

AGENT-RELEASING VASCULAR PROSTHESIS

PROTHESE VACSULAIRE A LIBERATION DE SUBSTANCES ACTIVES

(84) Benannte Vertragsstaaten:
**AT BE BG CH CY CZ DE DK EE ES FI FR GB GR HU IE IT LI LU MC NL PL PT RO SE SI SK TR**

(30) Priorität: **19.09.2003 DE 10343922**
**20.11.2003 DE 10354359**

(43) Veröffentlichungstag der Anmeldung:
**07.06.2006 Patentblatt 2006/23**

(73) Patentinhaber: **Julius-Maximilians-Universität Würzburg**
**97070 Würzburg (DE)**

(72) Erfinder:
• **LARENA-AVELLANEDA, Axel**
**97286 Sommerhausen (DE)**
• **SIEGEL, Rolf**
**97076 Würzburg (DE)**
• **DEBUS, Eike Sebastian**
**21075 Hamburg (DE)**

(74) Vertreter: **Bohmann, Armin K.**
**bohmann**
**Anwaltssozietät**
**Nymphenburger Strasse 1**
**80335 München (DE)**

(56) Entgegenhaltungen:
**WO-A1-02/03890     WO-A1-90/13332**
**WO-A1-96/08149**

• **LARENA-AVELLANEDA A ET AL: "The silicone coated polyester prosthesis. Further modifications and introduction of the "drug releasing graft"" GEFASSCHIRURGIE 2004 GERMANY, Bd. 9, Nr. 2, 2004, Seiten 105-110, XP002314930 ISSN: 0948-7034**
• **DATABASE WPI Section Ch, Week 198701 Derwent Publications Ltd., London, GB; Class A96, AN 1987-003571 XP002314933 & JP 61 263448 A (HIROYOSHI H) 21. November 1986 (1986-11-21)**
• **DEBUS E S ET AL: "Silicone-covered polyester prosthesis. Results following subrenal aortic repair in a dog model" GEFASSCHIRURGIE 2002 GERMANY, Bd. 7, Nr. 2, 2002, Seiten 65-69, XP002314931 ISSN: 0948-7034**
• **CHEN C ET AL: "Phosphorylcholine coating of ePTFE grafts reduces neointimal hyperplasia in canine model." ANNALS OF VASCULAR SURGERY. JAN 1997, Bd. 11, Nr. 1, Januar 1997 (1997-01), Seiten 74-79, XP002314932 ISSN: 0890-5096**
• **OKKEMA A Z ET AL: "Bulk, surface and blood-contacting properties of polyether polyurethanes modified with polydimethylsiloxane macroglycols." BIOMATERIALS. JAN 1989, Bd. 10, Nr. 1, Januar 1989 (1989-01), Seiten 23-32, XP000007968 ISSN: 0142-9612**

**Beschreibung**

Die vorliegende Erfindung betrifft eine Prothese .

Gefäßprothese sind in der modernen Therapie bei einer Vielzahl von Erkrankungen, wie z.B. Gefäßaussakkungen, Gefäßeinengungen oder Verschlüssen bei arterieller Verschlusskrankheit / Diabetes mellitus, und chronischem Nierenversagen, ein wichtiges Hilfsmittel für den Arzt, insbesondere dann, wenn krankheitsbedingt eine Gefäßrekonstruktion erforderlich ist und diese Gefäßrekonstruktion nicht durch Zugriff auf autologes Gefäßmaterial möglich ist, da dieses entweder krankhaft verändert ist, oder bereits entfernt wurde. Im Stand der Technik werden in derartigen Fällen Kunststoffprothesen verwendet. Infolge ihrer Anwendung als Gefäßersatz ist es günstig, wenn die Gefäßprothesen primär dicht sind. Unter dem Begriff Gefäßprothesen versteht man verschiedene Formkörper wie Rohre, Y-Prothesen oder Patches, die mit biologischem Material in Kontakt kommen. Rohre haben zum einen ein proximales Ende und ein distales Ende die durch einen sich dazwischen erstreckenden Hohlraum miteinander verbunden sind, wobei der Hohlraum die Aufnahme eines Fluids erlaubt, ohne dass das Fluid aus dem Hohlraum durch die Wand der Prothese dringt bzw. Flüssigkeiten von außerhalb der Gefäßprothese nach innen in den Hohlraum dringen. Y-Prothesen sind Y-förmig verzweigte Rohre, die zwei proximale Enden und ein distales Ende besitzen und durch ein sich dazwischen erstreckender Hohlraum miteinander verbunden. Der Hohlraum erlaubt die Aufnahme eines Fluids, ohne dass das Fluid aus dem Hohlraum durch die Wand der Prothese dringt bzw. Flüssigkeiten von außerhalb der Gefäßprothesen nach innen in den Hohlraum dringt. Y-Prothesen und Rohre haben zwei Kontaktflächen, eine Innenwand im Hohlkörper zum Blut hin und eine Außenwand des Hohlkörpers zum Gewebe hin.

Im Gegensatz dazu besitzen Patches, oder auch Flickenplastiken, keinen Hohlraum, sie sind zweidimensional, wie beispielsweise ein Lappen, und besitzen zwei Kontaktflächen, die mit biologischem Gewebe in Berührung kommen.

Im Stand der Technik wurde die Verwendung von gewirkten Polyesterprothesen als Gefäßprothesen beschrieben. Derartige gewirkte Polyesterprothesen sind jedoch primär undicht. Entsprechend werden sie bevorzugterweise in einen Zustand überführt, der diese Undichtigkeit beseitigt. Dies wird typischerweise dadurch erreicht, dass die Polyesterprothesen mit Kollagen, Albumin oder Gelatine vom Rind beschichtet werden, die jedoch den Nachteil aufweisen, dass sie tierische Produkte in den menschlichen Körper einfahren, was mit Blick auf bovine spongioforme Encephalopathie (BSE) auch unter regulatorischen Aspekten zunehmend weniger akzeptiert wird.

Ein im Stand der Technik weiterhin verwendetes Trägermaterial für Prothesen ist expandiertes Polytetrafluoroethylen (PTFE), welches jedoch dazu neigt, bei Implantation zu Stichkanalblutungen zu führen, was durch die Beschichtung mit Materialien wie Kollagen, Albumin oder Gelatine mit den zuvor beschriebenen nachteiligen Wirkungen zu verhindern versucht wird.

Schließlich weisen die im Stand der Technik beschriebenen, aus Kunststoff hergestellten Gefäßprothesen allgemein noch den Nachteil auf, dass sie biologisch nicht inert sind, d.h. im Laufe der Zeit abgebaut werden, dass ihre Oberflächen oftmals thrombogen sind und dass sie zur Ausbildung einer Intimahyperplasie, insbesondere an den Anastomosen, führen.

Bei der Intimahyperplasie handelt es sich um eine Reaktion der Gefäße auf eine Verletzung (Larena-Avellaneda et al. 2004, Weiterführende Modifikation und Entwicklung des "drugeluting bypass", Gefäßchirurgie, im Druck). Es kommt zur Vermehrung von glatten Muskelzellen in der Media der Arterie. Diese wandern in die Intimaschicht ein und beginnen mit der Produktion von sogenannter extrazellulärer Matrix.

Diese mit der Verwendung derartiger Gefäßprothesen einhergehenden Nachteile stellen neben dem Fortschreiten der Grundkrankheiten, die zur Implantation derartiger Prothesen führen, den wesentlichen Grund dar, weshalb Kunststoffprothesen nach dem Stand der Technik nicht die von medizinischer Seite gewünschte und erforderliche Wirkung aufweisen. Unter Inertheit versteht man, dass die Prothese nach Implantation nicht abgebaut wird. Unter Thrombogenität wird dabei die Eigenschaft einer Gefäßprothese verstanden, bei Kontakt derselben mit Blut die Ausbildung eines Thrombus und/oder dessen Ablagerung zu induzieren. Die Entwicklung der Intimahyperplasie geht nach derzeitigem Verständnis auf eine Verletzung der Endothelien zurück, was zur Anlagerung von Thrombozyten führt. Diese und auch die Gefäßzellen selbst produzieren Wachstumsfaktoren, wie beispielsweise PDGF, FGF-2, Endothelin I, Angiotensin II und Matrixmetalloproteinasen [Bauters C. et al., Cardiovasc.Res. 31:835-846; Ben Slimane S. et al., Eur.Surg.Res. 20:12-17], die dann wiederum zusammen mit anderen Einflüssen, wie beispielsweise Hormonen und mechanischen Faktoren, über die Veränderung der glatten Muskelzellen zur Intimahyperplasie führten, wobei bereits 30 Minuten nach einer Gefäßverletzung eine Aktivierung der glatten Muskelzellen nachzuweisen ist. 24 bis 48 Stunden später beginnen die glatten Muskelzellen in der Media zu proliferieren. Nach vier Tagen kommt es zu einer Migration der glatten Muskelzellen in die Intima und nach einer Woche zeigt sich eine maximale Proliferationsrate der glatten Muskelzellen. Diese hält für einige Wochen an, wobei zusätzliche extrazelluläre Matrix mit Kollagen vom Kollagentyp I/III, Elastin, Fibronectin und Proteoglycan produziert wird. Ein Gleichgewichtszustand wird nach ca. drei Monaten erreicht. Die Intima besteht dann zu 47 bis 80 % aus der extrazellulären Matrix. Es kommt sekundär zu einer Infiltration der Gefäßwand durch Monozyten, T-Zellen und anderen Leukozyten aus dem Plasma.

Im Stand der Technik sind verschiedene Ansätze beschrieben, um die Intimahyperplasie zu unterdrücken.

Diese umfassen beispielsweise die Bestrahlung der Anastomosen [Soni A. B. et al., Int.J.Radiat.Oncol.Biol.Phys. 54: 1174-1179], gentherapeutische Ansätze [Conte M. S. et al., J.Vasc.Surg. 36:1040-1052] und Tissue Engineering [Teebken O. E. et al., Eur.J.Vasc.Endovasc.Surg. 23:475-485] sowie die Beschichtung des Implantates mit Endothelzellen [Arts C. H. et al., Eur.J.Vasc.Endovasc.Surg. 23:29-38; Conte M. S. et al., J.Vasc.Surg. 21:413-421; Gomes D. et al., J.Vasc.Surg. 34:707-715; Seifalian A. M. et al., Artif.Organs 26:307-320]. Die bisher angewandten Beschichtungen, wie z.B. Carbon im Falle des Produktes Carboflo® der Fa. Impra oder Heparin im Falle des Produktes Propaten® der Fa. Gore haben klinisch keine durchgreifenden Erfolge gezeigt [Imig, H Grundmann, RT, 2000, Gefäßprothesen - Wo geht es hin?, Zentralbl. Chir. 125: 298). Ein entscheidender Nachteil dieser Gefäßprothesen liegt beispielsweise bei dem Produkt Propaten in der fehlenden lokalen Freisetzung des Wirkstoffs Heparin, der irreversible an die Prothese gebunden ist. Auf der Innenseite der Prothese ragen aktive Sequenzen, der an die Prothese gebundenen Heparinmoleküle ins Lumen und binden Antithrombin, wobei Antithrombin durch die Bindung so konformiert wird, dass es in der Lage ist besonders rasch Thrombin zu binden. Der Thrombinantithrombinkomplex wird durch den Blutfluss vom Heparinmolekül weggespült. Durch die Bildung des Komplexes soll die Entstehung eines Thrombus verhindert werden. Auch bei dem Produkt Carboflo® wird der Wirkstoff Carbon nicht freigesetzt, sondern wirkt nur als bioaktive Beschichtung. Auch diese Oberflächenbeschichtung soll bewirken, dass die Prothese weniger thrombogen ist.

All diesen Ansätzen ist jedoch gemein, dass sie in der Regel mit einem hohen Aufwand an Material und Zeit verbunden sind bzw. sich noch in einer sehr frühen Phase der Entwicklung befinden und ihre Anwendung mehr experimenteller Natur denn täglich geübte klinische Praxis ist.

Der vorliegenden Erfindung liegt somit die Aufgabe zugrunde, eine Prothese bereitzustellen, die die Nachteile der Prothesen nach dem Stand der Technik überwindet. Insbesondere ist es eine Aufgabe der vorliegenden Erfindung, eine dichte Prothese bereitzustellen, die nach Implantation wesentlich länger im Körper verweilt als herkömmliche Prothesen. Erfindungsgemäß ist die Prothese biologisch inert, weist eine geringe Thrombogenität und eine geringe Neigung zur Ausbildung einer Intimahyperplasie nach Implantation auf.

Diese und andere der vorliegenden Erfindung zugrunde liegende Aufgaben werden durch den Gegenstand der beigefügten unabhängigen Ansprüche gelöst. Bevorzugte Ausführungsformen ergeben sich aus den abhängigen Ansprüchen.

Genauer wird die der vorliegenden Erfindung zugrunde liegende Aufgabe in einem ersten Aspekt, der auch die erste Ausführungsforin des ersten Aspektes ist gelöst, durch eine Prothese umfassend ein proximales und ein distales Ende mit einem sich dazwischen erstreckenden Hohlraum, wobei die Prothese eine Trägerstruktur und mindestens eine biologisch aktive Verbindung umfasst, die Trägerstruktur aus einem Trägermaterial und einem Beschichtungsmaterial besteht, wobei das Beschichtungsmaterial Polydimethylsiloxan ist, wobei die Beschichtung der Prothese 10 - 30 mg Polydimethylsiloxan/cm$^2$ Oberfläche beträgt.

In einer zweiten Ausführungsform des ersten Aspektes, die auch eine Ausführungsform der ersten Ausführungsform des ersten Aspektes ist, beträgt die Beschichtung der Prothese 15 - 26 mg Polydimethylsiloxan/cm$^2$ Oberfläche.

In einer dritten Ausführungsform des ersten Aspektes, die auch eine Ausführungsfbrm der zweiten Ausführungsform des ersten Aspektes ist, beträgt die Beschichtung der Prothese 16 - 23 mg Polydimethylsiloxan/cm$^2$ Oberfläche.

In einer vierten Ausführungsform des ersten Aspektes, die auch eine Ausführungsform der ersten, der zweiten und der dritten Ausführungsform des ersten Aspektes ist, ist das Trägermaterial ausgewählt aus der Gruppe, die Polytetrafluoroethylen, Polyester und Polyurethan umfasst.

In einer fünften Ausführungsform des ersten Aspektes, die auch eine Ausführungsform der ersten, der zweiten, der dritten und der vierten Ausführungsform des ersten Aspektes ist, bilden das Trägermaterial und das Beschichtungsmaterial eine Matrix aus.

In einer sechsten Ausführungsform des ersten Aspektes, die auch eine Ausführungsform der fünften Ausführungsform des ersten Aspektes ist, enthält die Matrix die biologisch aktive Verbindung.

In einer siebten Ausführungsform des ersten Aspektes, die auch eine Ausführungsform der ersten, der zweiten, der dritten, der vierten, der fünften und der sechsten Ausführungsform des ersten Aspektes ist, definiert die Trägerstruktur eine Außenwand und eine Innenwand, wobei die Innenwand den Blut führenden Hohlraum und die Außenwand die Trägerstruktur gegen das umgebende Gewebe begrenzt.

In einer achten Ausführungsform des ersten Aspektes, die auch eine Ausführungsform der siebten Ausführungsform des ersten Aspektes ist, ist die Prothese flüssigkeitsdicht.

In einer neunten Ausführungsform des ersten Aspektes, die auch eine Ausführungsform der ersten, der zweiten, der dritten, der vierten, der fünften, der sechsten, der siebten und der achten Ausführungsform des ersten Aspektes ist, weist das Beschichtungsmaterial eine Modifikation auf.

In einer zehnten Ausführungsform des ersten Aspektes, die auch eine Ausführungsform der neunten Ausführungsform des ersten Aspektes ist, ist die Modifikation aus der Gruppe ausgewählt, die Silanolat, Alkylhydroxid, Polyvinylalkohol, Polyethylenoxid, Zinksulfat, Polyvinylpyrrolidon und Phosphocholin umfasst.

In einer elften Ausführungsform des ersten Aspektes, die auch eine Ausführungsform der zehnten Ausführungsform des ersten Aspektes ist, ist die Modifikation aus der Gruppe ausgewählt, die Polyvinylalkohol, Polyvinylpyrrolidon und Phosphocholin umfasst.

In einer zwölften Ausführungsform des ersten Aspektes, die auch eine Ausführungsform der neunten, der zehnten und der elften Ausführungsform des ersten Aspektes ist, ist die Modifizierung an der Innenwand vorhanden und verbleibt bevorzugterweise dort dauerhaft.

In einer dreizehnten Ausführungsform des ersten Aspektes, die auch eine Ausführungsform der ersten, der zweiten, der dritten, der vierten, der fünften, der sechsten, der siebten, der achten, der neunten, der zehnten, der elften und der zwölften Ausführungsform des ersten Aspektes ist, ist mindestens eine biologisch aktive Verbindung in der Matrix vorhanden.

In einer vierzehnten Ausführungsform des ersten Aspektes, die auch eine Ausführungsform der ersten, der zweiten, der dritten, der vierten, der fünften, der sechsten, der siebten, der achten, der neunten, der zehnten, der elften, der zwölften und der dreizehnten Ausführungsform des ersten Aspektes ist, ist die biologisch aktive Verbindung aus der Gruppe ausgewählt, die Antibiotika, Immunsuppressiva und Proliferationshemmer umfasst.

In einer fünfzehnten Ausführungsform des ersten Aspektes, die auch eine Ausführungsform der ersten, der zweiten, der dritten, der vierten, der fünften, der sechsten, der siebten, der achten, der neunten, der zehnten, der elften, der zwölften, der dreizehnten und der vierzehnten Ausführungsform des ersten Aspektes ist, ist die biologisch aktive Verbindung aus der Gruppe ausgewählt, die Acetylsalicylsäure, Sirolimus und Paclitaxel umfasst.

In einer sechszehnten Ausführnmgsform des ersten Aspektes, die auch eine Ausführungsform der ersten, der zweiten, der dritten, der vierten, der fünften, der sechsten, der siebten, der achten, der neunten, der zehnten, der elften, der zwölften, der dreizehnten, der vierzehnten und der fünfzehnten, insbesondere der achten, der neunten, der zehnten, der elften, der zwölften, der dreizehnten, der vierzehnten und der fünfzehnten Ausführungsform des ersten Aspektes ist, wird die biologisch aktive Verbindung mit einer Rate von 2 - 3 $\mu$g/Stunde abgegeben.

In einer siebzehnten Ausführungsform des ersten Aspektes, die auch eine Ausführungsform der ersten, der zweiten, der dritten, der vierten, der fünften, der sechsten, der siebten, der achten, der neunten, der zehnten, der elften, der zwölften, der dreizehnten, der vierzehnten, der fünfzehnten und der sechszehnten Ausführungsform des ersten Aspektes ist, ist die Prothese eine Gefäßprothese.

In einer achtzehnten Ausführungsform des ersten Aspektes, die auch eine Ausführungsform der ersten, der zweiten, der dritten, der vierten, der fünften, der sechsten, der siebten, der achten, der neunten, der zehnten, der elften, der zwölften, der dreizehnten, der vierzehnten, der fünfzehnten, der sechszehnten und der siebzehnten Ausführungsform des ersten Aspektes ist, ist die Prothese bei der kniegelenksübergreifenden alloplastischen Rekonstruktion verwend bar.

In einer neunzehnten Ausführungsform des ersten Aspektes, die auch eine Ausführungsform der ersten, der zweiten, der dritten, der vierten, der fünften, der sechsten, der siebten, der achten, der neunten, der zehnten, der elften, der zwölften, der dreizehnten, der vierzehnten, der fünfzehnter, der sechszehnten, der siebzehnten und der achtzehnten Ausführungsform des ersten Aspektes ist, ist die Prothese als pheripherer oder zentraler AV-Shunt verwendbar.

In einer zwanzigsten Ausführungsform des ersten Aspektes, die auch eine Ausführungsform der ersten, der zweiten, der dritten, der vierten, der fünften, der sechsten, der siebten, der achten, der neunten, der zehnten, der elften, der zwölften, der dreizehnten, der vierzehnten, der fünfzehnten, der sechszehnten, der siebzehnten, der achtzehnten und der neunzehnten Ausführungsform des ersten Aspektes ist, wird die Prothese als Patch oder als Dialyse-Shunt verwendbar.

In einer noch bevorzugteren Ausführungsform ist das Trägemiaterial Polyester.

Auch offenbart wird ein Verfahren zur Herstellung einer Prothese umfassend ein proximales und ein distales Ende mit einem sich dazwischen erstreckenden Hohlraum, wobei die Prothese eine Trägerstruktur und mindestens eine biologisch aktive Verbindung umfasst und die Trägerstruktur aus einem Trägermaterial und einem Beschichtungsmaterial besteht, insbesondere eine Prothese gemäß dem ersten Aspekt der vorliegenden Erfindung, umfassend die Schritte:

- Bereitstellen des Trägermaterials, wobei das Trägermaterial ein proximales Ende und ein distales Ende und einen sich dazwischen erstreckenden Hohlraum umfasst;

- Beschichten des Trägermaterials mit einem Beschichtungsmaterial, wodurch eine Matrix ausgebildet wird; und

- Einbringen der biologisch aktiven Verbindung in die Matrix.

Auch offenbart wird, dass die Beschichtung des Trägermaterials erfolgt, indem das Beschichtungsmaterial auf das rotierende Trägermaterial aufgegeben wird.

Auch offenbart wird, dass der Beschichtungsschritt mehrfach wiederholt wird und bevorzugterweise nach jedem Beschichtungsschritt das aufgebrachte Beschichtungsmaterial oder das beschichtete Trägermaterial getrocknet wird.

Auch offenbart wird, dass das Beschichtungsmaterial bereits eine Modifikation aufweist.

Auch offenbart wird, dass das Beschichtungsmaterial und/oder die Matrix mit einer Modifikation, bevorzugterweise an der Oberfläche und bevorzugtererweise an der dem Hohlraum zugewandten Wand der Trägerstruktur, versehen und damit modifiziert wird.

Auch offenbart wird, dass das Modifizieren durch einen nass-chemischen Modifizierungsvorgang erfolgt.

Auch offenbart wird, dass die Modifikation durch ein Molekül oder eine dadurch bereitgestellte Gruppe ausgebildet wird, die ausgewählt ist aus der Gruppe, die Polyvinylalkohol, Phosphocholin und Polyvinylpyrrolidon umfasst.

Auch offenbart wird, dass vor dem nass-chemischen Modifizierungsvorgang eine Aktivierung der Matrix mittels basischer Methanollösung erfolgt.

Auch offenbart wird, dass nach der Aktivierung das die Modifikation ausbildenden Molekül in einer verdünnten Lösung mit der Matrix, bevorzugterweise mit der Innenwand der Matrix und/oder der Trägerstruktur kontaktiert wird.

Auch offenbart wird, dass die biologisch aktive Verbindung durch einen nass-chemischen Vorgang in die Matrix eingebracht wird.

Auch offenbart wird, dass vor oder während des Einbringens der biologisch aktiven Verbindung die Matrix durch Kontaktieren der Matrix mit einem Quellmittel aufgequollen wird.

Auch offenbart wird, dass die biologisch aktive Verbindung in einem Lösungsmittel bereitgestellt wird und das die biologisch aktive Verbindung enthaltende Lösungsmittel mit der Matrix in Kontakt gebracht wird.

Auch offenbart wird, dass das Lösungsmittel ein Quellmittel für die Matrix ist.

Auch offenbart wird, dass das Trägermaterial um seine Längsachse, die sich durch oder parallel zum Hohlraum des Trägermaterials erstreckt, gedreht wird, während das Beschichtungsmaterial auf das Trägermaterial aufgegeben wird.

Auch offenbart wird, dass das Beschichtungsmaterial als Präpolymer bereitgestellt wird und während oder nach dem Kontaktieren mit dem Trägermaterial polymerisiert.

Auch offenbart wird, dass das Beschichtungsmaterial vom distalen Ende zum proximalen Ende oder vom proximalen Ende zum distalen Ende aufgegeben wird, wobei das Trägermaterial relativ zu dem aufgegebenen Beschichtungsmaterial in Richtung der Längsachse des Trägermaterials bewegt wird.

Auch offenbart wird, dass das Trägermaterial aus Polyester und das Beschichtungsmaterial ist Polydimethylsiloxan besteht.

Auch offenbart wird, dass das Trägermaterial mit etwa 80 Umdrehungen/Minute gedreht wird.

Auch offenbart wird, dass das Beschichtungsmittel eine Lösung von etwa 11 g des Präpolymers von Polydimethylsiloxan in etwa 86 g Ethylacetat ist.

Auch offenbart wird, dass die relative Bewegung etwa 14 mm/Minute und die Tropfgeschwindigkeit beträgt etwa 15 -20 ml/Stunde: beträgt.

Auch offenbart wird, dass ein Beschichtungsdurchgang beendet, ist wenn das Trägermaterial einmal von seinem proximalen Ende bis zu seinem distalen Ende oder von seinem distalen Ende bis zu seinem proximalen Ende beschichtet worden ist.

Auch offenbart wird, dass der Beschichtungsvorgang mehrfach wiederholt wird.

Auch offenbart wird, dass eine Prothese herstellbar nach einem Verfahren wie hierin offenbart.

Der vorliegenden Erfindung liegt die überraschende Erkenntnis zugrunde, dass bei einer Prothese, die eine Trägerstruktur und mindestens eine biologisch aktive Verbindung umfasst, gleichzeitig die Thrombogenität und die durch die Prothese bedingte Intimahyperplasie in ihrer Ausprägung verringert bzw. vermieden werden kann. Die Trägerstruktur der erfindungsgemäßen Prothese besteht dabei aus einem Trägermaterial und einem Beschichtungsmaterial. Zur Vermeidung der mit der Verwendung von tierischem und insbesondere bovinem Material, wie beispielsweise Kollagen, Albumin oder Fibrinogen, verbundenen Risiken, wie sie beispielsweise BSE darstellt, handelt es sich bei dem Beschichtungsmaterial bevorzugterweise um einen Kunststoff. Der vorliegende Erfinder hat dabei überraschenderweise festgestellt, dass als Kunststoff besonders Organopolysiloxane geeignet sind. Durch die Verwendung eines derartigen Kunststoffes als Beschichtungsmaterial ist es möglich, dass verschiedene Trägermaterialien eingesetzt werden, wie sie als solche im Stand der Technik für die Herstellung von Prothesen oder Stents verwendet werden. Ein besonders bevorzugtes Ausgangsmaterial stellen insoweit Polytetrafluoroethylen und Polyester, wie beispielsweise Polyethylenterephthalat, oder Polyurethan dar. Es ist im Rahmen der vorliegenden Erfindung, dass das Trägermaterial allgemein aus solchen Materialien hergestellt sein kann, die als Blutleiter in Betracht kommen.

Die erfindungsgemäße Prothese ist multifunktionell, d.h. durch Einbringung von biologisch wirksamen Molekülen auf die Oberfläche oder in die Matrix der Prothesen werden diese lokale Wirkungen zu entfalten, d. h. z.B. optimale Einheilung der Prothesen bei gleichzeitiger Entzündungsprophylaxe und /oder Thromboseprophylaxe. Die biologisch aktive Verbindung wird bevorzugterweise kontinuierlich an den Kontaktflächen, innen und außen, der Prothesen freigesetzt.

Die erfindungsgemäße Prothese ist somit bevorzugterweise eine rohrförmige Gefäßprothese. Neben der Verwendung als Gefäßprothese kann die erfindungsgemäße Prothese auch zur Rekonstruktion von gefäßähnlichen Strukturen, wie beispielsweise Körperpassagen oder - durchgängen, verwendet werden.

In einer bevorzugten Ausführungsform stellt die erfindungsgemäße Prothese einen Formkörper zum Gefäßersatz dar, der bevorzugterweise bei langstreckigen Verschlüssen verwendet wird.

Die erfindungsgemäße Prothese stellt einen Formkörper dar, der zwei Kontaktflächen zu biologischem Material besitzt. In der bevorzugten Form besteht der Formkörper aus einem Rohr, d.h. einem verlängerten Element mit einem proximalen und einem distalen Ende und einem sich dazwischen erstreckenden Hohlraum, das hierin auch als Trägerstruktur bezeichnet wird. Der Hohlraum definiert den Bereich, in dem die Körperflüssigkeit nach dem Einführen oder der Implantation der Prothese geleitet werden soll. Die Flussrichtung der Flüssigkeit ist dabei im Wesentlichen parallel zu der durch den Hohlraum definierten Längsachse der Prothese. Die Flussrichtung kann jedoch auch hiervon abweichen, wie beispielsweise der Fall bei extraanatomischen Umleitungen, Herzbypass und Dialyse Shunts. Dabei ist es bevorzugt, dass der Flüssigkeitsstrom durch den Hohlraum erfolgt und eine Passage über die den Hohlraum definierenden oder begrenzenden Prothesenwand nicht erfolgt, zumindest nicht wesentlich erfolgt. Eine Passage des Flüssigkeitsstroms ist jedoch beispielsweise erlaubt bei der Verwendung der erfindungsgemäßen Prothese als pheripherer oder zentraler AV-Shunt wie Dialyse-Shunt bzw. Carotis-Patch. Die Prothese wird somit von einer eine Prothesenwand aufweisende Trägerstruktur ausgebildet, wobei die Prothesenwand eine Außenwand und eine Innenwand definiert und die Innenwand den Hohlraum der Prothese begrenzt. Die erfindungsgemäße Prothese kann somit auch als Bypass bezeichnet oder verwendet werden. Die erfindungsgemäße Prothese kann darüber hinaus auch in Form eines Patches ausgebildet sein, somit eine im Wesentlichen zweidimensionale Struktur aufweisen, wobei sie zwei Kontaktflächen aufweist, die mit biologischem Gewebe in Berührung gelangen. Infolge der zweidimensionalen Ausbildung des Patches wird die Trägerstruktur die beiden Kontaktflächen ausbilden und der Patch keinen Hohlraum wie bei der rohrförmigen oder Y-förmigen Ausführungsform der erfindungsgemäßen Prothese aufweisen.

Bevorzugterweise handelt es sich bei der Prothese um einen Fremdkörper, d. h. einen Körper, der verschieden ist von dem Material, das in dem Organismus vorhanden ist, in den die Prothese implantiert wird, verschieden ist. Bevorzugtererweise handelt es sich bei der Prothese um eine solche, die von einem allogenen oder autologen Gefäßimplantat verschieden ist.

In einer weiteren Ausführungsform ist vorgesehen, dass die Trägerstruktur durch ein faseriges oder flächiges Material definiert ist. In einer weiteren Ausführungsform ist vorgesehen, dass die Trägerstrukturen aus einem gestrickten, gewirkten oder gewebten Material hergestellt ist.

Die Trägerstruktur umfasst das Trägermaterial und das Beschichtungsmaterial. Das Beschichtungsmaterial ist bevorzugterweise ein Organopolysiloxan. Aus der Gruppe der Organopolysiloxane sind insbesondere Polyvinylsiloxan, Polyphenylsiloxan und Polyalkylsiloxane bevorzugt. Wie hierin verwendet, bezeichnet der Begriff Alkyl eine geradkettige oder verzweigtkettige Kohlenwasserstoffkette, die eine oder mehrere Substituenten aufweisen kann. Bevorzugterweise weist die Kohlenstoffkette keine Substituenten auf. In einer bevorzugten Ausführungsform ist die Länge der Alkylkette ein bis sechs Kohlenstoffatome lang, bevorzugterweise ein bis vier Kohlenstoffatome lang und bevorzugtererweise ein bis zwei Kohlenstoffatome lang. Ein besonders bevorzugtes Polyalkylsiloxan ist dabei Polydimethylsiloxan, insbesondere additionsvernetzende Silikontypen. Die dreidimensionale Vernetzung von linearen Polydimethylsiloxan-Ketten führt zum fertigen Formkörper aus Silikon. Die Beschichtung von Trägermaterialien oder Trägerstrukturen mit Silikonen und insbesondere mit Siloxanen ist, beispielsweise, beschrieben von Rochow, E., (Eugene Rochow, Silcon and Silicones, Springer Verlag, ISBN 3-540-17565-2).

Die Beschichtung mit den Organopolysiloxanen stellt eine wirksame Möglichkeit dar, das Trägermaterial, welches bevorzugterweise nicht flüssigkeitsdicht ist, flüssigkeitsdicht auszugestalten. Ein Test zur Beurteilung der Flüssigkeitsdichtigkeit ist im Beispielsteil beschrieben. Flüssigkeitsdicht ist eine Prothese gemäß der vorliegenden Erfindung bei Werten von ≤5 ml/Min./cm$^2$ bei einem Druck innerhalb des Gefäßes von 120 mmHg.

Es ist im Rahmen der vorliegenden Erfindung, dass das Trägermaterial und das Beschichtungsmaterial zusammen eine Matrix ausbilden. Es ist jedoch auch im Rahmen der vorliegenden Erfindung, dass die Matrix durch das Beschichtungsmaterial oder das beschichtete Trägermaterial aufgebaut ist.

Die erfindungsgemäße Prothese umfasst in einer Ausführungsform eine oder mehrere Modifikationen. Diese Modifikationen dienen, ohne im Folgenden darauf festgelegt sein zu wollen, wohl ganz besonders Beeinflussung der Thrombogenität der erfindungsgemäßen Prothese. Die Modifikation wird bevorzugterweise als eine Modifikation der Matrix, bevorzugterweise als eine Oberflächenmodifikation der Matrix ausgeführt. Wie hierin verwendet bezeichnet Oberflächenmodifikation eine Modifikation, die an einer Oberfläche vorhanden ist. Bevorzugterweise handelt es sich bei der Oberfläche um jene, die mit der im Hohlraum der Prothese enthaltenen oder dadurch strömenden Flüssigkeit in Kontakt gelangt. Eine derartige Modifizierung der Matrix, d. h. ein Versehen der Matrix mit einer oder mehreren Modifikationen, kann vor der Verwendung des Beschichtungsmaterials erfolgen, d. h. bevor das Trägermaterial mit dem Beschichtungsmaterial beschichtet wird. In einem solchen Fall trägt das Beschichtungsmaterial bereits die Modifikation. Alternativ kann eine Modifizierung, insbesondere der Matrix, vorgenommen werden, nachdem das Trägermaterial mit dem Beschichtungsmaterial beschichtet worden ist. Die Modifikation kann dabei durch die folgenden Moleküle bzw. die von diesen Molekülen bereitgestellten reaktiven Gruppen ausgebildet werden: Alkylhydroxid, Polyvinylalkohol (PVA), Polyethylenoxid (PEO), Zinksulfat, Silanolat, Polyvinylpyrrolidon (PVP) und Phosphocholin (PC). Bevorzugterweise ist der Alkylrest der Alkylhydroxide wie hierin allgemein für Alkyl definiert.

Im Sinne einer eine besonders geringe Thrombogenität aufweisende oder bedingende Modifikationen haben sich Polyvinylalkohol (PVA), Phosphocholin (PC) und die PDMS-Oberfläche erwiesen.

In einer Ausführungsform der erfindungsgemäßen Prothese ist vorgesehen, dass die biologisch aktive Verbindung in der Matrix enthalten ist oder an der Oberfläche gebunden vorhanden ist. Dabei ist es im Rahmen der vorliegenden Erfindung, dass die biologisch aktive Verbindung an zumindest einer der Kontaktflächen der Prothese und/oder in der Matrix, d. h. in dem Bereich der Trägerstruktur zwischen der Außenwand und der Innenwand, vorhanden ist. Es ist im Rahmen der vorliegenden Erfindung ist, dass die biologisch aktive Verbindung sowohl an der Innenwand, d. h. der dem Hohlraum zugewandten Seite der Trägerstruktur vorhanden ist, als auch an der Außenwand. Es ist somit ganz besonders bevorzugt, wenn die biologisch aktive Verbindung mit dem die Prothese nach Implantation umgebenden Gewebe oder einer derartigen Flüssigkeit und auch mit der im Hohlraum der Prothese enthaltenen oder dadurch hindurchströmenden Flüssigkeit in Kontakt gebracht wird.

Die biologisch aktive Verbindung wird entweder kovalent oder ionogen an der Matrix gebunden. Bevorzugterweise wird die biologisch aktive Verbindung an oder in der Matrix adsorptiv immobilisiert.

Die biologisch aktive Verbindung ist bevorzugterweise aus der Gruppe ausgewählt, die Antibiotika, Immunsuppressiva und Proliferationshemmer umfasst. Insbesondere die Verwendung von Immunsuppressiva oder Proliferationshemmern dient entscheidend dazu, die unerwünschte Intimahyperplasie zu unterdrücken. Ganz besonders bevorzugt sind dabei Sirolimus und Paclitaxel. Weitere bevorzugte Verbindungen sind Abciximab, Östrogen, Thrombozytenaggregationshemmer wie ASS, Vitamin K- Antagonisten wie Coumarine, Everolimus, Tacrolimus und Wachstumsfaktoren. Es ist im Rahmen der vorliegenden Erfindung, dass auch zwei oder mehrere biologisch aktive Verbindungen von der Prothese umfasst und insbesondere in oder auf der Matrix enthalten sind. Die zwei oder mehreren biologisch aktiven Verbindungen können dabei gleichzeitig oder zeitlich versetzt abgegeben werden Beispiele hierfür sind bei entzündlichen Prozessen Immunspressivum und Antibiotikum. Weitere bevorzugte biologisch aktive Verbindungen sind beispielsweise zur Thromboseprophylaxe: Thrombozytehaggregationshemmer oder Vitamin-K-Antagonisten oder Heparine, zur verbesserten Funktionalität der Prothese: Wachstumsfaktoren mit lokaler und peripherer Wirkung, zur Entzündungshemmung; Corticosteroide und Hormone, wie z. B. Östrogene, zur Verhinderung von Hyperplasien; monoklonale Antikörper, zur Verhinderung von Abstoßungsreaktionen; Immunmodulatoren wie Cytokine, oder Enzyme. Es können auch genetisch veränderte Zellen und Zellbestandteile, enzymhemmende Proteine, Peptide verwendet werden. Möglich ist auch die Verwendung von retardierten Stoffen, um zeitverzögerte Wirkungen der Stoffe hervorzurufen. Dabei können die Stoffe chemisch verändert, oder galenisch verpackt sein. Eine retardierte Form von Sirolimus stellt beispielsweise Rapamycin-28-N,N-dimethylglycinatmethansulfonsäure dar. Beschichtete Stents mit einer langsam eluierenden Form von Sirolimus zeigten in Studien eine geringere Intimahyperlasie- und Restenoserate gegenüber Stents, die mit einem schnell eluierenden Form von Sirolimus ausgestattet waren (Carter, AL et al., 2004, Cardiovascular Res., 63, 617-624).

Infolge des in der Einleitung beschriebenen Mechanismus des Entstehens von Intimahyperplasie, der hierin durch Bezugnahme zur Vermeidung unnötiger Wiederholungen aufgenommen wird, ist dabei bevorzugt, dass für die biologisch aktive Verbindung ein definiertes Elutionsprofil realisiert wird, insbesondere mit Blick auf die Kinetik der Entstehung der Intimahyperplasie. Es wird im Rahmen des Könnens der Fachleute auf dem Gebiet sein, dass bei Verwendung unterschiedlicher pharmazeutisch oder biologisch aktiver Verbindungen ein geeignetes Elutionsprofil für die einzelne Verbindung der erfindungsgemäßen Prothese eingestellt wird. Dies kann unter Berücksichtigung der konkreten Gegebenheiten des Einzelfalls, wie Strömungsverhältnisse, Umfang an Flüssigkeitsstrom, Wirksamkeit der einzelnen Verbindung am Implantationsort und dergleichen festgelegt werden. Beispielsweise wird die Abgabe der einzelnen Verbindung im Bereich von 1 bis 10, bevorzugterweise 2 bis 3 µg/ml im Falle eines Immunsuppressivums IS sein. Es ist im Rahmen der vorliegenden Erfindung, dass die Fachleute auf dem Gebiet die Immobilisierung von biologisch aktiven Verbindung diese insbesondere so vornehmen, dass eine lokale Abgabe der Verbindung erfolgt. Dabei ist besonders bevorzugt, dass ein maximaler Wirkspiegel der biologisch aktiven Verbindung lokal vorhanden ist und der systemische Spiegel unter der minimal wirksamen Dosis liegt, um Nebenwirkungen zu vermeiden.

Gemäß der vorliegenden Erfindung wird die erfindungsgemäße Prothese insbesondere bei der kniegelenksüberschreitenden alloplastischen Rekonstruktion verwendet. Die Prothese kann generell verwendet werden für pheriphere und zentrale AV-shunts. Weitere Anwendungen der erfindungsgemäßen Prothese ist als Carotis-Patch, insbesondere als Carotis-Patch nach Desobliteration, als ACVB, d. h. aortocoronarer Venenbypass oder als implantierter Dialyse-Shunt zur Dialysebehandlung.

Auch hierin offenbart wird ein Verfahren zur Herstellung einer Prothese wobei die Prothese insbesondere eine solche Prothese ist, wie sie im ersten Aspekt der vorliegenden Erfindung hierin offenbart ist. Es wird anerkannt werden, dass sowohl die Merkmale und AusKhmngsforrnen der Prothese, wie sie im Zusammenhang mit dem hierin offenbarten Verfahren beschrieben sind, Merkmale der Prothese gemäß dem ersten Aspekt der vorliegenden Erfindung sein können, und umgekehrt, d. h. Merkmale und Ausgestaltungsformen einzeln und unabhängig von der weiteren Realisierung der für die konkret beschriebenen und hierin offenbarten Ausführungsform der Prothese, wie sie im Zusammenhang mit der erfindungsgemäßen Prothese gemäß dem ersten Aspekt der vorliegenden Erfindung offenbart ist, auch Merkmale und Ausfüluungsformen der durch das hierin offenbarte Verfahren hergestellten oder herstellbaren Prothese sind.

Das hierin offenbarte Verfahren zur Herstellung der besagten Prothese umfasst dabei die Schritte:

- Bereitstellen des Trägermaterials, wobei das Trägermaterial ein Formkörper ist, der zum Gefäßersatz dient

- Beschichten des Trägermaterials mit einem Beschichtungsmaterial, wodurch eine Matrix ausgebildet wird, und

- Einbringen der biologisch aktiven Verbindung in die Matrix oder auf die Oberfläche.

In Übereinstimmung mit dem ersten Aspekt der vorliegenden Erfindung kann das Trägermaterial ein Formkörper sein, wobei bevorzugterweise der Formkörper ausgewählt ist aus der Gruppe, die Rohre, Y-Prothesen und Patches umfasst.

Als Trägermaterial und Beschichtungsmaterial sind besonders jene Materialien geeignet, wie sie im Zusammenhang mit der erfindungsgemäßen Prothese hierin offenbart sind. Obwohl nicht darauf beschränkt, wird im Rahmen des hierin offenbarten Verfahrens das Beschichtungsmaterial auf das Trägermaterial aufgebracht, insbesondere durch Auftropfen, wobei das Trägermaterial um seine durch den Hohlraum definierte Längsachse rotiert. Bei der Rotation handelt es sich bevorzugterweise um eine gleichmäßige Rotation, was insoweit von Vorteil ist, als dass damit eine gleichmäßige Beschichtung in Umfangsrichtung gewährleistet wird. Das verwendete Beschichtungsmaterial kann dabei ein Beschichtungsmaterial sein, das die Modifikationen, wie im Zusammenhang mit der erfindungsgemäßen Prothese beschrieben, bereits aufweist. Alternativ ist es jedoch auch möglich, dass das Beschichtungsmaterial entsprechend nach dem Beschichten des Trägermaterials mit der Modifikation versehen wird. Es ist entsprechend hierin offenbart , dass die Modifikationen erst in die Matrix eingebracht werden.

In dem hierin offenbarten Verfahrens, bei dem die Modifikationen erst in die Matrix eingebracht werden, ist es sowohl im Rahmen des Verfahrens, dass das Einbringen der biologisch aktiven Verbindung in die Matrix nach der Modifizierung der Matrix, d. h. nach der Aufnahme der Modifikation(en) in die Matrix erfolgt, wie auch vorher, d. h. dass die biologisch aktive Verbindung in die Matrix eingebracht wird, noch bevor die Modifikation(en) in die Matrix eingebracht werden.

Der Beschichtungsvorgang wird bevorzugterweise durch Verwendung des Beschichtungsmaterials in seinem nicht vollständig polymerisierten Zustand durchgeführt. Dabei ist vorgesehen, dass ein Präpolymer oder eine Vorform des Polymers auf das Trägermaterial aufgebracht wird und die Ausbildung des Polymers nach dem Aufbringen entweder unmittelbar oder zeitversetzt erfolgt. Das Beschichtungsmaterial kann auf das Trägermaterial gemäß den Fachleuten auf dem Gebiet bekannten Techniken aufgebracht werden. Dazu gehören, u. a. Tauchverfahren, Sprühverfahren oder Auftropfverfahren, wobei Auftropfverfahren, wie in den Beispielen detaillierter beschrieben, besonders bevorzugt sind. Das Auftropfverfahren eignet sich insbesondere bei Herstellungsbedingungen die flüssige bis zähe Beschichtungsmaterialien verwenden.

Es ist weiterhin im Rahmen des Verfahrens, dass der Beschichtungsvorgang mehrfach wiederholt werden kann. Bevorzugterweise ist das Ende der Wiederholung des Beschichtungsvorganges dann erreicht, wenn die Prothese die erwünschte Dichtigkeit aufweist, die erforderliche Wandstärke erreicht wird, die erwünschten Elastizitätseigenschaften, wie sie für die im Einzelfall geplante Anwendung erforderlich ist, von der Prothese aufgewiesen werden, und/oder wenn die Prothese in dem Umfang mit dem Beschichtungsmaterial beschichtet ist, dass ein Abbau der Prothese im Organismus, in dem die Prothese implantiert werden soll, nicht mehr oder mit einer deutlich verringerten Geschwindigkeit erfolgt oder erfolgen kann.

Es ist dabei im Rahmen des Verfahrens, dass die Ausbildung des Polymers nach einem jeden Einzelschritt erfolgt, bevorzugterweise durch Trocknen bei bevorzugterweise erhöhten Temperaturen. In Abhängigkeit von dem jeweils verwendeten Beschichtungsmittel, insbesondere dem Polyalkylsiloxan, wie beispielsweise Polydimethylsiloxan), sind dabei Temperaturen von 60 bis 90°, bevorzugterweise 75° bevorzugt, wobei die Auswahl im Rahmen der Fähigkeiten der Fachleute auf dem Gebiet erfolgt und, unter anderem, die Stabilität der verwendeten Substanzen berücksichtigt. Es wird anerkannt werden, dass Beschichtungsmittel, die auf der Grundlage eines anderen Polymerisationsmechanismus aushärten, entsprechend andere Bedingungen benötigen, die sodann bevorzugterweise nach einem jeden Einzelschritt realisiert werden.

Der Modifizierungsvorgang, d. h. der Vorgang der Vornahme des Einbauens der Modifikationen in die Matrix, ist bevorzugterweise als nass-chemischer Vorgang ausgerührt, wobei die die Modifikationen bereitstellende Verbindung bzw. das dazu korrespondierende Molekül in einer verdünnten Lösung bereitgestellt wird. Bevorzugterweise ist die verdünnte Lösung unter Verwendung von unpolare Lösungsmittel wie beispielsweise Heptan oder Hexan als Lösungsmittel hergestellt.

Bevorzugterweise wird vor dem Kontaktieren der die Modifikation bereitstellenden Moleküle enthaltenden Lösungen die Matrix aktiviert. Diese Aktivierung dient dazu, die Modifikationen entsprechend in die Matrix oder auf die Oberfläche aufzunehmen. Die Aktivierung erfolgt bevorzugterweise durch eine basische Methanollösung.

Die in der Prothese enthaltene biologisch aktive Verbindung wird bevorzugterweise ebenfalls durch einen nass-chemischen Vorgang in die Matrix eingebracht. Bevorzugterweise erfolgt dabei die Immobilisierung der biologisch aktiven Verbindung auf der Grundlage eines physikalischen Bindungsmechanismus.

In einer bevorzugten Ausführungsform ist die biologisch aktive Verbindung somit an die Matrix, bevorzugterweise an das Beschichtungsmaterial, adsorptiv gebunden und kann von oder aus dieser eluiert werden, so dass es sich bei der erfindungsgemäßen Prothese um eine die biologisch aktive Verbindung eluierende Prothese handelt. Für eine besonders effektive Immobilisierung ist dabei vorteilhaft, dass vor oder während des Einbringens der biologisch aktiven Verbindung in die Matrix die Matrix gequollen wird. In Abhängigkeit von dem verwendeten Trägermaterial und insbesondere dem verwendeten Beschichtungsmaterial können hierzu verschiedene Quellmittel verwendet werden. Bevorzugterweise wird die biologisch aktive Verbindung als Lösung bereitgestellt, wobei das Lösungsmittel bevorzugterweise ein oder das Quellmittel ist.

Die Erfindung wird nun anhand der folgenden Zeichnungen und Beispiele weiter erläutert, aus denen sich weitere Merkmale, einzeln oder in Kombination, Ausführungsformen und Vorteile der Erfindung ergeben. Dabei zeigt:

Fig. 1 eine schematische Darstellung des Herstellungsprozesses der erfindungsgemäßen Prothese;

Fig. 2 die für Oberflächenmodifizierung verwendeten Moleküle, die hierin auch als Modifikation bezeichnet werden, wobei die grundsätzlichen chemischen Strukturelemente, die wesentlichen Eigenschaften hinsichtlich der Thrombogenität und Literaturstellen, die die entsprechenden Modifikationen beschreiben, angegeben sind;

Fig. 3 rasterelektronenmikroskopische Aufnahmen von verschiedenen Prothesen, wobei

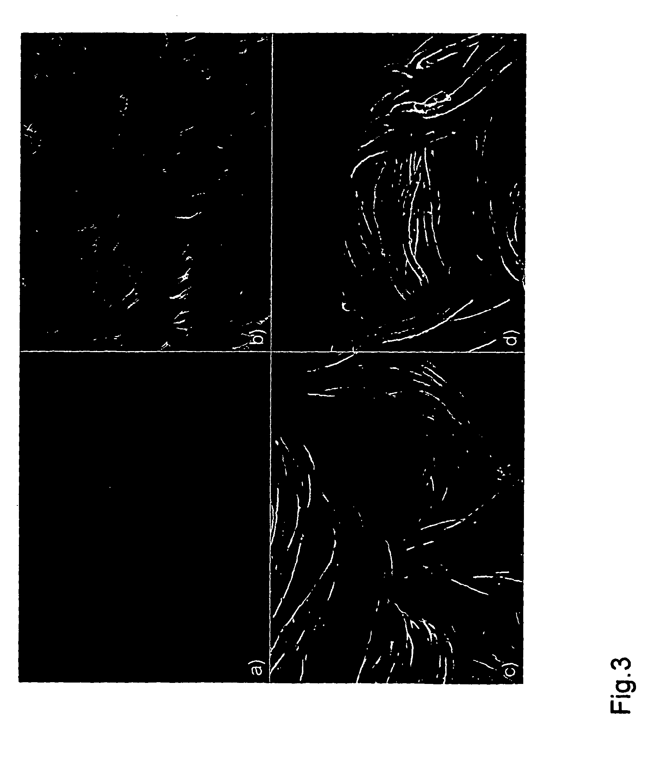

Fig. 3 a) eine Kollagen-beschichtete Polyester-Prothese nach dem Stand der Technik ist;

Fig. 3 b) eine PTFE-Prothese nach dem Stand der Technik ist;

Fig. 3 c) die erfindungsgemäße Prothese mit einer Beschichtung von 11 mg Polydimethylsiloxan /cm$^2$ Prothesen-oberfläche; und

Fig. 3 d) eine erfindungsgemäße Prothese mit einer Beschichtung von 20 mg Polydimethylsiloxan/cm$^2$ Prothesen-oberfläche;

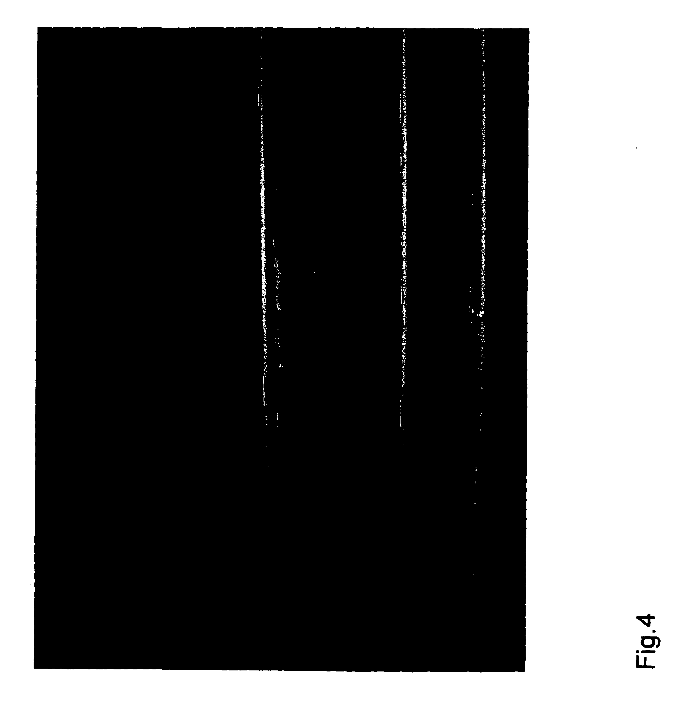

Fig.4 eine Darstellung der Blutverteilungsmuster in den verschiedenen oberflächenmodifizierten Prothesen nach einer Stunde Rotationszeit, wobei die netzartige Verteilung bei Prothese A (Silonolat), E (Zinksulfat), F (PDNS) und H (Phosphocholin) zu erkennen ist;

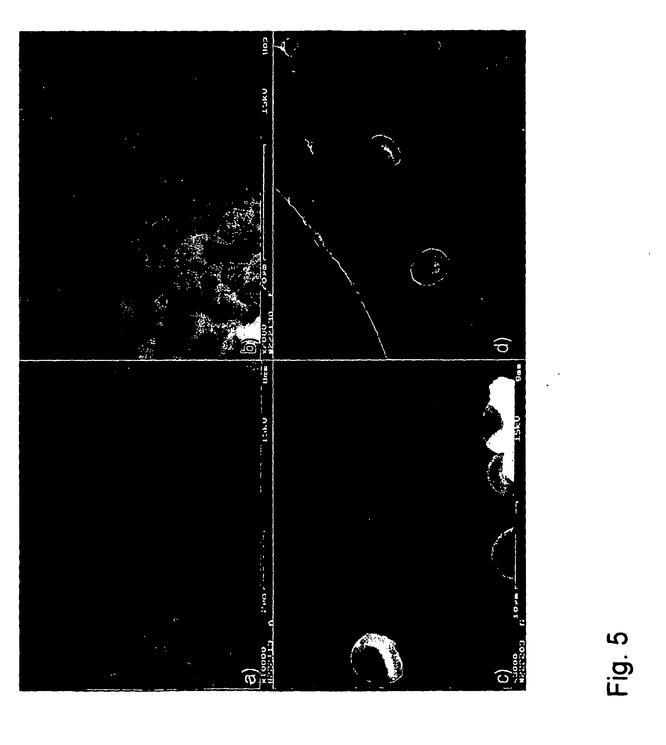

Fig. 5 eine rasterelektronenmiskropische Aufnahme verschiedener Prothesen nach Kontakt mit Blut;

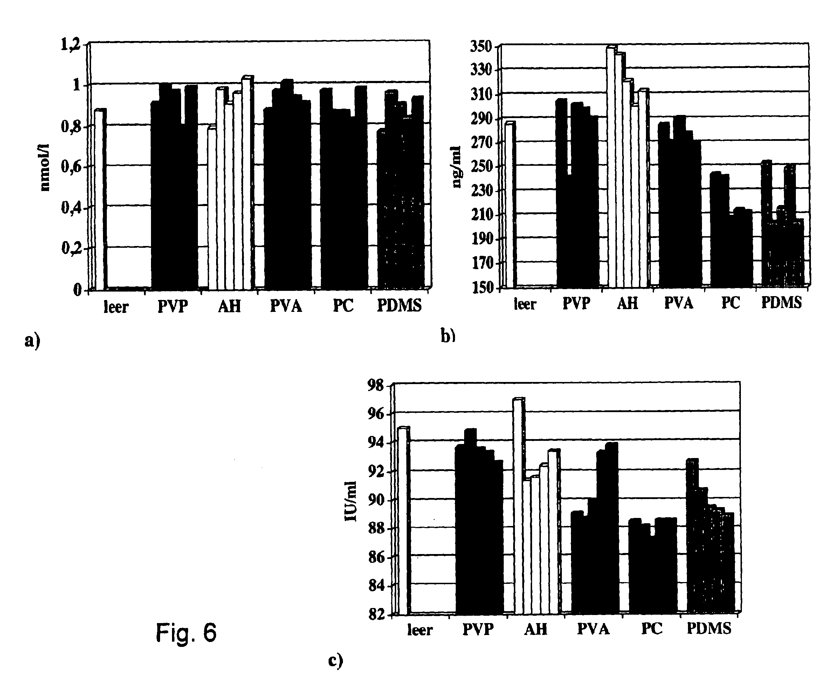

Fig. 6 das Ergebnis der Bestimmung der Gerinnungsparameter bei Verwendung verschiedener Prothesen, wie im Rahmen von ELISA-Analysen ermittelt;

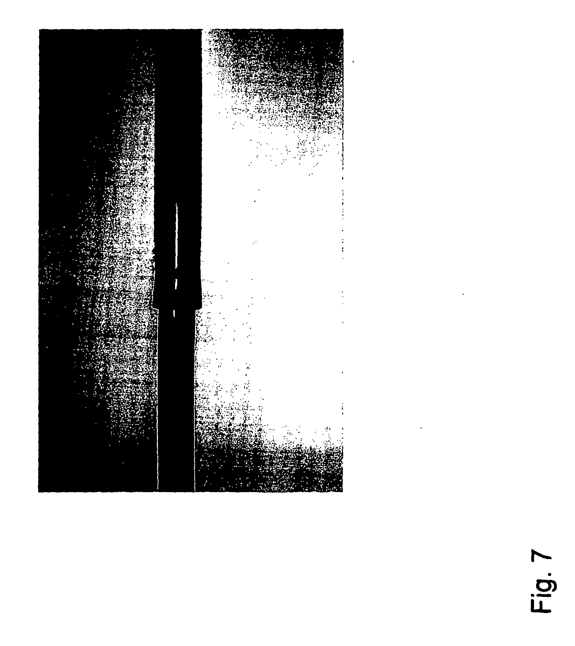

Fig. 7 die Verteilung von Sudanrot im Bereich der Kontaktflächen im Rahmen der Untersuchungen der Elution von Molekülen im Kreislaufmodell;

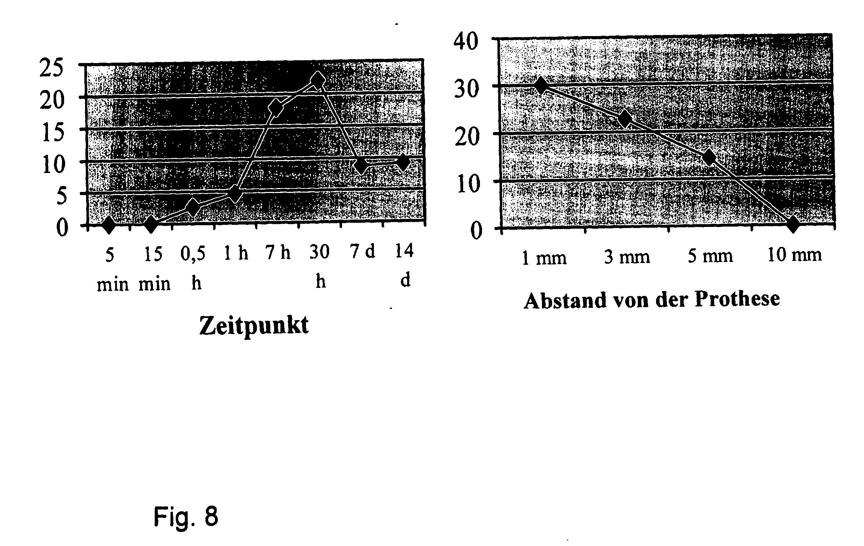

Fig. 8 a) eine graphische Darstellung der Freisetzungskinetik des Immunsuppressivums IS in Medium;

Fig. 8 b) die Freisetzung des Immunsuppressivums IS aus einer in einer Rinderarterie implantierten Prothese gemäß der vorliegenden Erfindung als Funktion des Abstandes von der Prothese;

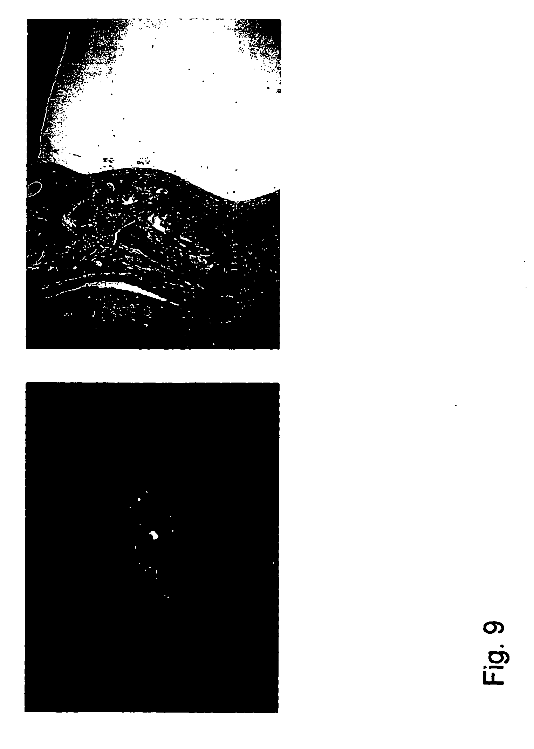

Fig. 9 a) eine photographische Abbildung einer eine erfindungsgemäße Prothese enthaltenden Iliacagabel;

Fig. 9 b) eine histologische Darstellung der Prothese

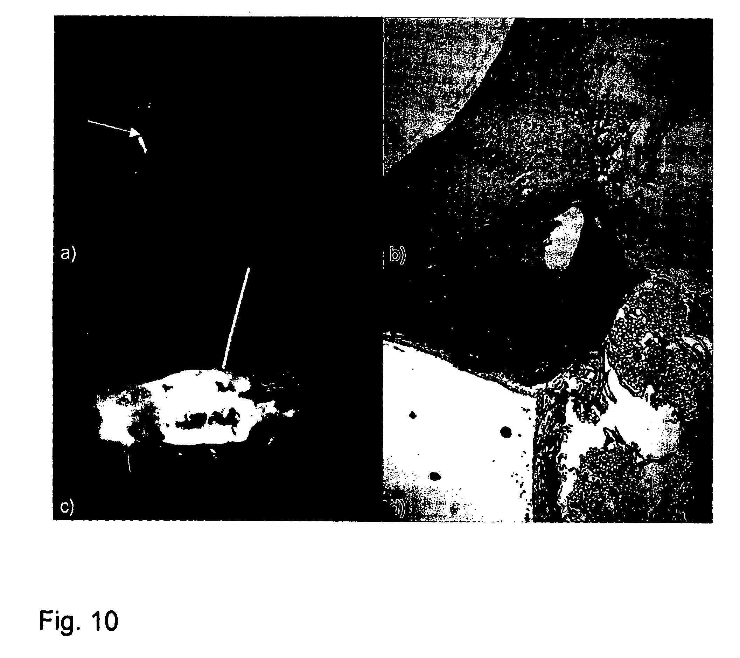

Fig. 10 explantierte Ilicagabeln zusammen mit Prothesen nach dem Stand der Technik (Fig. 10 a) und b)) und mit Prothesen gemäß der vorliegenden Erfindung (Fig. 10 c) und d));

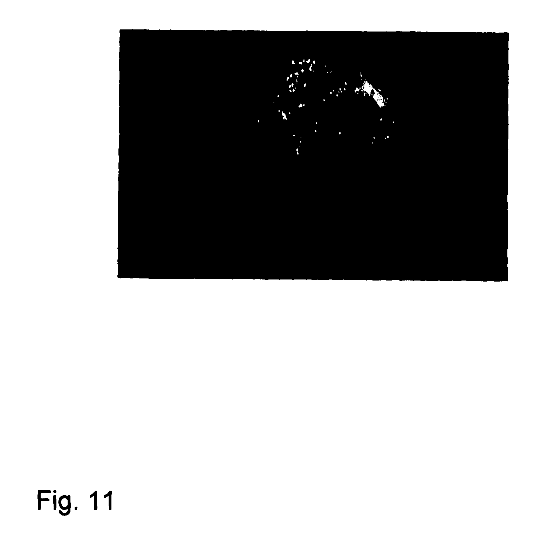

Fig. 11 eine photographische Darstellung einer explantierten Ilicagabel nach Implantation einer Prothese gemäß der vorliegenden Erfindung als Patchplastik, wobei die Prothese Paclitaxel umfasste.

**Beispiel 1: Herstellung einer Prothese**

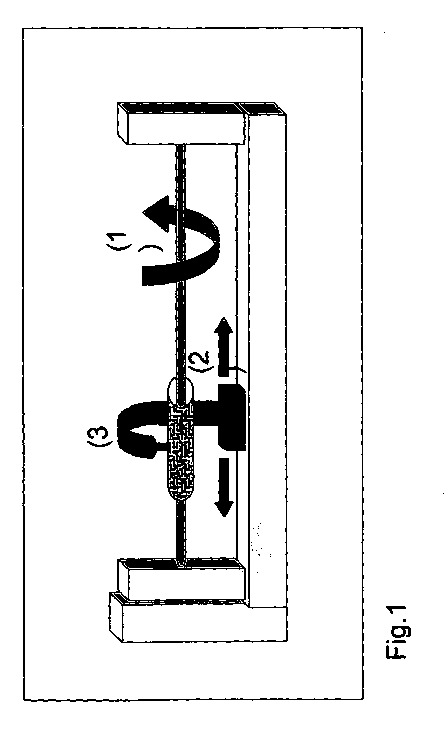

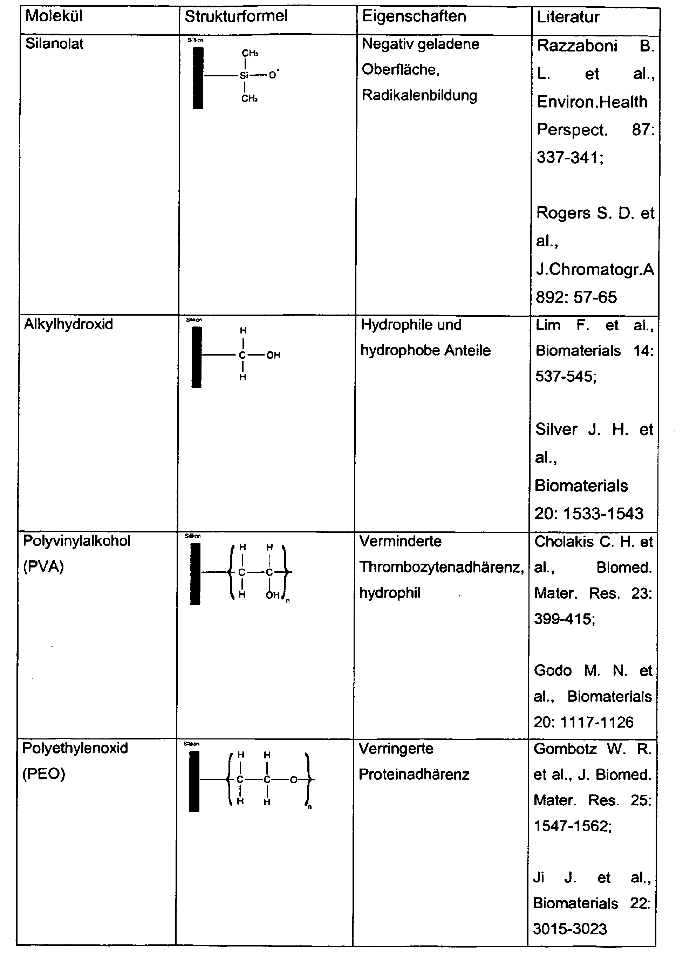

Es wurde eine Prothese mit einem Durchmesser von 6 mm und einer Länge von 200 mm (Micron™, Firma Intervascular) aus gewirktem Polytethylenerephthalat bereitgestellt. Die als Ausgangsmaterial verwendete Prothese stellt somit die Trägerstruktur der erfindungsgemäßen Prothese dar. Die Trägerstruktur wurde sodann wie in Fig. 1 dargestellt durch den ihre Längsachse definierenden Hohlraum auf einen Rotationsstab aufgebracht und dieser Rotationsstab in eine sich drehende Produktionsvorrichtung eingespannt. Der Rotationsstab dreht sich mit einer definierten Geschwindigkeit um die eigene Längsachse (1). Die in Fig. 1 dargestellte Produktionsvorrichtung umfasst eine Dosiervorrichtung (3), die das Beschichtungsmittel bereitstellt. Im vorliegenden Falle handelt es sich bei dem Beschichtungsmittel um das Präpolymer von Polydimethylsiloxan, nämlich MDX4-4210 der Fa. Dow Corning für Tierversuche, beim Menschen implant grade, beispielsweise von den Firmen NuSil oder Applied Silicone, das als eine Lösung von 11 g des Präpolymers von Polydimethylsiloxan in 86 g Ethylacetat verwendet wurde. Die Dosiervorrichtung (3) selbst ist parallel zur Längsachse des Hohlraumes und zu dem rotierenden Stab (1) an der Produktionsvorrichtung befestigt. Aus einer Düse der Dosiervorrichtung (3) tropft das Präpolymer auf das Trägermaterial und beschichtet dieses. In Umfangsrichtung wird das Präpolymer auf das Trägermaterial dadurch aufgebracht, dass sich das Trägermaterial auf dem rotierenden Stab (1) befindet. In Abhängigkeit von der Breite der Austrittsöffnung der Düse und deren Streuwinkel bzw. Austrittswinkel wird ein dazu korrespondierender breiter Abschnitt des Umfanges des Trägermaterials mit dem Präpolymer beschichtet. Es wird für die Fachleute auf dem Gebiet offensichtlich sein, dass die Breite der Düse und die Tropfgeschwindigkeit die Stärke der durch das Auftropfen aufgebrachten Beschichtungsmittelschicht definiert. Im vorliegenden Falle betrug die Breite der Düse 0.9 mm.

Die Ausbildung einer Prothese mit einem Gehalt von 20 bis 22 mg Silikon/cm$^2$ Oberfläche, insbesondere der geometrischen Oberfläche des Trägermaterials wurde dadurch erreicht, dass das Trägermaterial mit 80 Umdrehungen/Minute gedreht wurde. Der Vorschub der Dosiervorrichtung (3) in Richtung (2) betrug dabei 14 mm/Minute. Die Tropfgeschwindigkeit betrug 20 ml/Stunde im ersten Durchgang der Beschichtung. Der Prozess wurde insgesamt dreimal wiederholt, wobei die Tropfgeschwindigkeit im zweiten Durchgang der Beschichtung (18) ml/Stunde und im dritten Durchgang der Beschichtung 17 ml/Stunde betrug. Zwischen den einzelnen Beschichtungsvorgängen wurde das Silikon zum Aushärten auf 75˚C erhitzt.

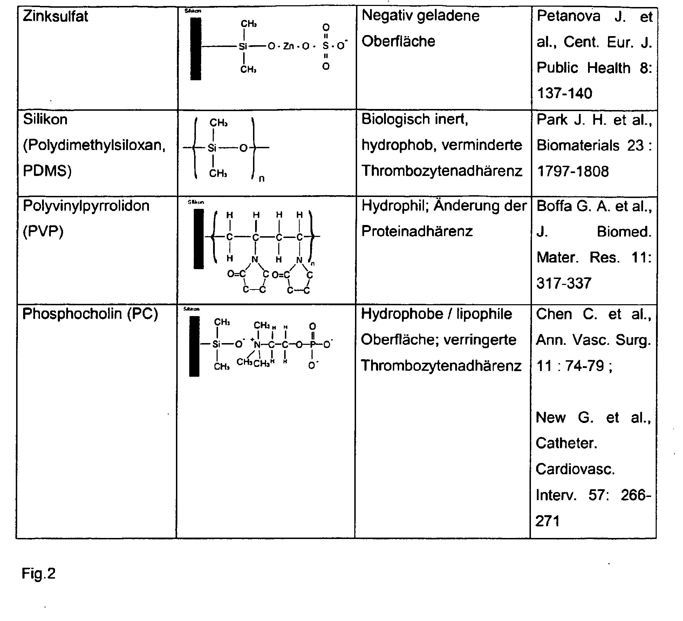

In einem zweiten Schritt wurde die Oberflächenmodifikation nass-chemisch eingeführt. Grundsätzlich sind die in Fig. 2 dargestellten Modifikationen geeignet, um auf bzw. in die aus dem Trägermaterial und Beschichtungsmaterial ausgebildete Matrix eingebracht zu werden.

Die Ergebnisse, die mit den unterschiedlichen Modifikationen erreicht wurden, werden in den nachfolgenden Beispielen im einzelnen diskutiert werden.

Die Oberflächenmodifizierung wurde dabei so vorgenommen, dass die Matrix durch Behandlung mittels basischer Methanollösung (5,7 Gew.-% KOH für 12 min) und mehrmaligem Auswaschen mit deionisiertem Wasser, dann durch Behandlung mit 0,5 Gew.-% PVP-Lösung (Luniquat PQ 11 PN der Fa. BASF, Leverkusen) für 120 min und anschließend durch Behandlung mit mehrmaligem Waschen aktiviert wurde. Danach wurden verdünnte Lösungen der verschiedenen in Fig. 2 dargestellten Moleküle verwendet, um die solchermaßen aktivierte Matrix zu kontaktieren und die Modifikation in die Matrix einzuführen.

In einem weiteren Schritt wurde sodann die biologisch aktive Verbindung eingebracht. Die Verbindungen wie bspw. ASS, Gentamycin, Paclitacel, Serolimus, ein Immunsuppressivum IS werden physikalisch in die Matrix des Silikons dadurch inkorporiert, dass man die mit ausgehärtetem Silikon beschichtete Prothese für 15 Stunden mit einer 0,05 Gew. %igen acetonischen Lösung mit den aktiven Verbindungen kontaktiert, danach mit Aceton wäscht und dann über Nacht bei Raumtemperatur trocknet, wobei die durch das Aceton gequollene Silikonschicht wieder ihre ursprüngliche Form annimmt. Dabei handelt es sich um einen adsorptiven Vorgang, was die nachfolgende Freigabe der biologisch aktiven Verbindung nach Implantation der Prothese erlaubt.

Für die Anwendung wurden die Prothesen plasmasterilisiert und steril verpackt.

**Beispiel 2: Verwendete technische Hilfsmittel**

Für die verschiedenen Untersuchungen, die Gegenstand der nachfolgenden Beispiele sind, wurden die folgenden technischen Hilfsmittel verwendet. Es ist dabei für die Fachleute offensichtlich, dass ähnliche Instrumente grundsätzlich verwendet werden können.

Das Gewicht der Prothesen zur Errechnung des Silikongehaltes wurde mit einer Präzisionwaage ermittelt. Die rasterelektronenmikroskopische Beurteilung erfolgte nach üblicher Vorbereitung im Zentrum für Elektronenmikroskopie der Universität Würzburg bei 50- 5000-facher Vergrößerung. Die Untersuchungen hinsichtlich der Stabilität / Elastizität wurden im Fadenzuggerät bzw. Tensiometer vorgenommen (Instron 4502, Klemmbackenabstand 2 cm, Zug / Druckgeschwindigkeit 50mm/ min), die Kraft-Weg-Diagramme wurden anhand eines Computers erstellt (Software Series IX, Fa. Instron). Um den Berstungsdruck zu ermitteln wurde ein Ballon-Katheter (Ultrathin™Diamond™, 12mm, Fa. Boston-Scientific) mit Hilfe eines Inflators/Druckmessers verwendet (Medflator II, Medex Medical, Lancashire, GB). Die Permeabilitäts- und Compliancemessungen wurden mit Hilfe eines Kreislaufmodells realisiert. Als Grundlage diente ein Kreislauf aus 8mm Silikonschläuchen (Fa. Rehau, Rehau). Das Modell wurde geschlossen oder offen mit 2 Zulauf/ Ablaufrohren angewendet. Eine Zirkulation wurde durch eine Turbinenpumpe erreicht (12 V Pumpe, Fa. Comet). Druck und Fluss wurden kontinuierlich gemessen (Druckmesser: GMH 3110, Fa. Greisinger, Flowmesser: DFM/POM + ARS 260 Totaliser, Fa. B.I.O. Tech). Standardmäßig wurde ein Fluss von 500ml/min bei einem Druck von 120mmHg eingestellt. Wir konnten weiterhin die exakten Innendurchmesser der Grafts mit Hilfe eines intravasculären Ultraschallgerätes ausmessen (IVUS; Gerät: Clear-view™Ultra, freundlicherweise von der Fa. Boston Scientific zur Verfügung gestellt, Sonde: Sonicath™Ultra 20MHz, 5F, Meditech, Fa. Boston Scientific). Als Medium, soweit erforderlich, diente in allen Untersuchungen hinsichtlich der physikalischen Eigenschaften destilliertes Wasser. In Anlehnung an die ISO Norm wurden alle Analysen mit Ausnahme der Compliance Messung (37,2°C) bei Raumtemperatur vorgenommen.

**Beispiel 3: Physikalische Eigenschaften der Prothese**

Die Ergebnisse der Bestimmung der physikalischen Eigenschaften der gemäß Beispiel 1 hergestellten Prothese können dahingehend zusammengefasst werden, dass die Prothesen durch die Silikonbeschichtung nicht instabil werden. Die Vorteile der erfindungsgemäßen Prothese bestehen darüber hinaus vielmehr darin, dass, wie auch experimentell belegt, reaktive Moleküle an der Oberfläche der Trägerstruktur maskiert werden und die Prothese durch die Silikonbeschichtung nach Implantation nicht abbaubar ist.

Die Silikon-beschichtete Prothese, wie in Beispiel 1 hergestellt, wurde genauer hinsichtlich ihrer physikalischen bzw. mechanischen Eigenschaften untersucht. Zusätzlich zu den nach ISO Norm 7198 vorgeschriebenen Tests (s. Tabelle 2) wurden die Prothesen subjektiv beurteilt, rasterelektronenmikroskopisch untersucht und die Quer- und Längselastizität sowie der punktuelle Widerstand ermittelt. Neben den unbeschichteten Micron™Prothesen dienten zum Vergleich primär dichte 6 mm Implantate aus PTFE (Goretex®, Fa. Gore) sowie aus mit Kollagen beschichtetem Polyester (Intergard® knitted, Fa. Intervascular / Meadox knitted Hemashield®, Boston Scientific). Es wurden, ebenso in Anlehnung an die ISO Vorgaben, jeweils 3 Prothesen untersucht.

Die nachfolgende Tabelle 2 führt die ermittelten mechanischen Eigenschaften nach ISO 7198: 2-9, die verwendeten Instrumente, physikalischen Grundlagen und verwendeten Einheiten an. Dabei bezeichnet:

Compliance: D = Ausgangsdurchmesser der Prothese, $\Delta D$ die Änderung des Durchmessers bei der Änderung des Druckes ($\Delta P$). Elastizität: Es gilt das Hooke-sche Gesetz für elastische Verformungen. F = einwirkende Kraft, E = Elastizitätsmodul (charakteristisch für das jeweilige Material). 1 = Ausgangslänge der entspannten Prothese, $\Delta l$ = Längenänderung

Tabelle 2

| Messgröße | Device/Medium | Physikalische Grundlage/ Formel | Einheit |
|---|---|---|---|
| Silikongehalt pro cm$^2$ | Waage | Gewicht (beschichtete Prothese) / cm$^2$ - Gewicht (leere Prothese) / cm$^2$ | mg/cm2 |
| Wasserpermeabilität bei 120mmHg | Kreislaufinodell, destilliertes Wasser, Messbehälter | Menge ausgetretenes Wasser/Oberfläche der Prothese | ml/cm$^2$/min bei 120 mm Hg |
| Stabilität: zirkulär | Tensiometer | Tmax/2L | kN/mm |
| Stabilität: längs | Tensiometer | Tmax | kN |
| Berstungsdruck | Ballonkatheter | Max. Druck | MPa |
| Entspannter Innendurchmesser | Kreislaufmodell, IVUS, Wasser | Durchmesser bei gefüllter Prothese | mm |
| Durchmesser bei 120 mm Hg | Kreislaufmodell, IVUS, Wasser | Durchmesser bei 120 mm Hg | mm |

(fortgesetzt)

| Messgröße | Device/Medium | Physikalische Grundlage/ Formel | Einheit |
|---|---|---|---|
| Compliance (Dehnbarkeit) | Kreislaufmodell, IVUS, Wasser | $$C = \frac{\Delta D}{D \times \Delta P} \times 100\%$$ | %/mmHg* $10^{-2}$ |
| Elastizität: Längs | Tensiometer | Hooke-sches Gesetz $$F = \mathrm{E} \times \frac{\Delta l}{l}$$ | N |
| Elastizität: Quer | Tensiometer | Hookesches Gesetz | N |
| Punktueller Widerstand | Tensiometer, gerade Nadel (Maxon 2x0, Nadel GR65) | Erforderliche Kraft, um Material zu durchstoßen | N |

Die physikalischen Eigenschaften der Prothese können wie folgt zusammengefasst werden.

Silikongehalt:

Je nach Anzahl der Beschichtungsvorgänge steigen Silikongehalt und Wanddicke an. Die Werte liegen jedoch im Bereich von herkömmlichen Prothesen (Tabelle 3).

Tabelle 3: Gewicht und Wanddicke verschiedener 6mm-Prothesen pro $cm^2$ Oberfläche.

| Beschichtung: | Wanddicke (mm) | Gewicht (mg / $cm^2$) | Silikongehalt (mg / $cm^2$) |
|---|---|---|---|
| Unbeschichtet | 0,31 | 15,5 | |
| 1x Silikon | 0,33 | 26,6 | 11,1 |
| 2x Silikon | 0,35 | 32,1 | 16,6 |
| 3x Silikon | 0,43 | 38,1 | 22,6 |
| 4x Silikon | 0,46 | 41,4 | 25,9 |
| Meadox Hemashield® | 0,40 | 18,4 | |
| Intergard knitted® | 0,35 | 28,9 | |

Subjektive Beurteilung:

Die silikonisierte Polyesterprothese ist je nach Menge an Silikon unterschiedlich in ihrer Handhabung. Ab einem Gehalt von 30mg Silikon pro $cm^2$ imponiert sie als relativ steif. Auf der anderen Seite kann so in bestimmten Abschnitten eine höhere Stabilität erreicht werden. Bei 1- bis 2-facher Beschichtung (bis 20 mg / $cm^2$) entspricht die Prothese subjektiv in etwa einem herkömmlichen Implantat.

Rasterelektronenmikroskopie:

In der rasterelektronischen Untersuchung konnte eine gleichmäßige Verteilung des Silikons nachgewiesen werden (Fig. 3). Wurden die Beschichtungsvorgänge wiederholt, zeigte sich eine ebenso gleichmäßige Auskleidung mit dem PDMS.

Untersuchungen im Tensiometer (Stabilität / Elastizität

Im Tensiometer wurden die Längs- und Querstabilität sowie - Elastizität ermittelt, weiterhin der punktuelle Widerstand. Die Ergebnisse sind im Einzelnen in der Tabelle aufgeführt. Es zeigt sich, dass durch die Silikonbeschichtung sich die Stabilität der Prothese nicht ändert. Durch die Beschichtung wird die Kraft, die notwendig ist, das Material zu zerreißen, weder in Längs- noch Querrichtung verändert.

Demgegenüber steigt mit der Silikonmenge die Steifigkeit vor allem in Quer-, weniger auch in Längsrichtung an. Die ermittelte Kraft ergibt sich aus der Multiplikation der Dehnung mit dem Elastizitätsmodul. Da die Dehnung bekannt ist und die Kraft gemessen wird, kann dieses Modul für jedes hier untersuchte Material anhand der Hooke-schen Formel für elastische Verformungen eingeschätzt werden.

Die Quersteifigkeit korreliert dabei direkt mit der Silikonmenge (Tabelle 4). Die ist für die 2-, 3-und 4-fach Silikon beschichtete Prothese höher als für die übrigen Materialien. Die Längssteifigkeit hängt auch vom Crimping der Materialien ab. Da die verwendete Leerprothese ein relativ starkes Crimping aufweist, ist dementsprechend das Elastizitätsmodul gering. Insofern ist dieser Wert mit den Ergebnissen für die konventionellen Prothesen, die ein geringeres Crimping aufweist, nicht vergleichbar. Mit der Silikonmenge steigt dieser Wert geringer, offensichtlich erhöht das Silikon vor allem die Querstabilität..Die Kraft, die benötigt wird, die Prothese zu durchstechen ('punktueller Widerstand') ist überraschenderweise für die PTFE Prothese am geringsten, und steigt auch hier mit dem Grad der Silikonisierung. Untersuchungen in der eigenen Klinik hatten gezeigt, das eine Differenz von > 0,5 N subjektiv bei Durchstoß der Nadel wahrgenommen wird.

Die Kraft, die benötigt wird, die Prothese zu durchstechen ('punktueller Widerstand') ist überraschenderweise für die PTFE Prothese am geringsten, und steigt auch hier mit dem Grad der Silikonisierung. Untersuchungen in der eigenen Klinik hatten gezeigt, das eine Differenz von > 0,5 N subjektiv bei Durchstoß der Nadel wahrgenommen wird.

Tabelle 4

| Material | Quersteifigkeit / Elastizitätsmodul quer (N/mm$^2$) | Längssteifigkeit / Elastizitätsmodul längs (N/mm$^2$) | Punktueller Widerstand (N) | Berstungsdruck (atm) |
|---|---|---|---|---|
| Unbeschichtet | 0,58 | 0,14 | 1,62 | > 20 |
| 1x Silikon | 0,93 | 0,21 | 1,64 | > 20 |
| 2x Silikon | 1,16 | 0,23 | 1,93 | > 20 |
| 3x Silikon | 2,49 | 0,25 | 1,50 | > 20 |
| 4x Silikon | 3,19 | 0,27 | 2,21 | > 20 |
| Intergard® knitted | 0,8 | 0,54 | 1,87 | > 20 |
| PTFE | 0,98 | 11,91 | 1,36 | > 20 |

Untersuchungen im Kreislaufmodell:

Im Kreislaufinodell wurden die Compliance und die Dichtigkeit getestet. Die Ergebnisse sind in Tabelle 5 aufgeführt. Die unbeschichtete Micron™prothese verliert mehr als 1000ml pro Minute schon bei geringen Drucken. Aber schon ein Beschichtungsvorgang führt zu einer ausreichenden Dichtigkeit. Bei 16-30mg Silikon/cm$^2$ liegt der Wasserverlust bei 3ml/min/cm2 und damit im Bereich der normalerweise eingesetzten Prothesen (Tabelle 5). 3- und 4-fach beschichtete Prothesen sind komplett dicht.

Der Durchmesser der Prothesen im entspannten Zustand ändert sich nicht durch den Beschichtungsvorgang. Jedoch nimmt dieser bei zunehmender Silikonisierung bei 120 mm Hg ab, so dass eine geringere Dehnbarkeit, d. h. Compliance, resultiert. Auch hier liegen die Resultate für die 20-30 mg Silikon beschichteten Prothesen im Bereich der herkömmlichen. Erwartungsgemäß weist die PTFE-Prothese kaum eine Änderung des Durchmessers und damit eine geringe Compliance auf.

Tabelle 5: Physikalische Merkmale der untersuchten Materialien. Aufgeführt sind: Wasserpermeabilität und die Compliance der untersuchten Prothesen

| Material | Flüssigkeitsverlust (ml / cm$^2$ * min) | Compliance (%/mmHg*10$^{-2}$) |
|---|---|---|
| Unbeschichtet | > 1000 | n.u. |
| 1x Silikon | 680 | n.u. |
| 2x Silikon | 3,7 | 6,29 |
| 3x Silikon | 0 | 5,29 |
| 4x Silikon | 0 | 4,03 |

(fortgesetzt)

| Material | Flüssigkeitsverlust (ml / cm$^2$ * min) | Compliance (%/mmHg*10$^{-2}$) |
|---|---|---|
| Meadox Hemashield® | 2,9 | 5,77 |
| PTFE | 0 | 1,43 |

Berstungsdruck:

Mit dem 12 mm Ballon-Katheter gelang es nicht, die Polyesterprothesen zu sprengen. Die Beschichtungen auf dem Gewirk zeigten jedoch ab 4-5 atm Längsrisse. Nach maximaler Druckinflation auf 20 atm hatten die Prothesen allerdings ihre Elastizität verloren und behielten den Durchmesser von 12 mm. Die Menge an Silikon beeinflusste den Berstungsdruck nicht (Tabelle 4). Auch die PTFE-Prothese zerriss nicht.

**Beispiel 4: Bestimmung der biologischen Eigenschaften der Prothese**

Um die Thrombogenität von unterschiedlichen Oberflächenmodifikationen zu beurteilen, wurden zum Teil direkte, zum Teil indirekte Nachweismethoden eingesetzt. Eine direkte Beurteilung erfolgte zum einen makroskopisch, zum anderen rasterelektronenmikroskopisch. Der indirekte Nachweis wurde durch die Bestimmung von Blut- bzw. Gerinnungsparametern vorgenommen. Um eine ausreichende Oberflächenkontaktierung zu simulieren, wurde ein Modell etabliert. Hierin wurden die blutbefüllten Prothesen quer zur Längsachse bei einer Temperatur von 37˚C für bis zu 2 Stunden rotiert. Die Geschwindigkeit wurde so gewählt, dass eine Luftblase das ganze Rohr von einem Ende zum anderen abläuft. Somit wurde die komplette Oberfläche in jeder Prothese kontaktiert. Das Blut stammte in allen Versuchen vom gleichen Spender, die Antikoagulation war 30 Minuten vor Blutentnahme mit 3000 iE Heparin i.v. erfolgt.

Makroskopische Betrachtung der Fließeigenschaften: In der ersten Versuchsreihe wurden transparente Silikonschläuche verwendet. Die unterschiedlichen Charakteristika bei der Oberflächenbenetzung wurden festgehalten.

Rasterelektronenmikroskopische Begutachtung: Die Prothesen wurden vor und nach Blutkontakt rasterelektronenmikroskopisch untersucht

Allgemeine Gerinnung,sparameter: In den ersten Versuchen wurden folgende Parameter untersucht: Quick, pTT, TZ, AT III, Fibrinogen, Hb, Leukozytenzahl, Thrombozytenzahl.

ELISA-Analyse der Aktivatoren der Gerinnung / der Fibrinolyse sowie der Thrombozytenaktivierung: Ein weitergehende Untersuchung des Blutes nach Oberflächenkontaktierung wurde angeschlossen. Zunächst wurden die Proben zentrifugiert und als plättchenarmes Citratplasmas bei -20˚C aufbewahrt, später dann mit Hilfe des ELISA-Verfahrens analysiert:

Eine Aktivierung der Gerinnung wurde mit Hilfe des Prothrombinfragment F1+2, einem Spaltprodukt bei der Ausbildung von Thrombin, nachgewiesen (verwendetes Kit: Enzygnost® F+2 Micro, Fa. Dade-Behring). Als Nachweis der Aktivierung der Fibrinolyse gelten die D-Dimere (Asserachrom® D-Dimer, Fa. Roche-Diagnostics). Eine weitere Differenzierung der Gerinnung erfolgte im Hinblick auf die endogene = intrinsische bzw. exogene = extrinsische Aktivierung (Marker: Imubind® Factor XIIa, Fa. American diagnostica bzw. Tissue Factor (Imubind® Tissue factor, Fa. American diagnostica).

Der Nachweis der Thrombozytenaktivierung erfolgte mit Hilfe des Proteins ,β-Thromboglobulin' (Asserachrom® β-TG, Fa. Roche-Diagnostics). Diese Substanz befindet sich in den α-Granula der Plättchen, und wird bei Aktivierung derselben freigesetzt. Die Höhe des Spiegels korreliert mit dem Grad der Aktivierung.

Makroskonische Betrachtung der Fließeigenschaften: Bei der Betrachtung der Fließeigenschaften von Blut fielen für die verschiedenen Moleküle charakteristische Unterschiede auf: Hydrophobe Substanzen führten zu netzartigem, ruckartigem Fließen (s. Fig. 4, Tabelle 6), hydrophile Moleküle zu einem glatten Fluss.

Während bei PVA, PDMS und PVP keine Blutbestandteile haften blieben, klärte sich die Oberfläche der Prothesen nicht bei den übrigen Materialien. Bei Silanolat, PEO, Zinksulfat und Phosphocholin trat eine partielle Thrombose auf, bei Alkylhydroxid hatten sich Wandthromben ausgebildet. Die Polyester-Vergleichsprothese hatte minimale Wandthromben aufgewiesen.

Tabelle 6: Thrombenbildung in den verschiedenen Materialien nach 1 Stunde Rotation.

| Material | Thrombenbildung | Fließeigenschaften |
|---|---|---|
| A - Silanolat | Partialthrombus | netzartig |

(fortgesetzt)

| Material | Thrombenbildung | Fließeigenschaften |
|---|---|---|
| B - Alkylhydroxid | Wandthrombus | glatt |
| C - PVA | Kein Thrombus | Sehr glatt |
| D - PEO | Partialthrombus | Ruckartig |
| E - Zinksulfat | Partialthrombus | Netzartig |
| F - PDMS | Kein Thrombus | netzartig |
| G - PVP | Kein Thrombus | Sehr glatt |
| H - PC | Partialthrombus | netzartig |
| Polyester beschichtet | Minimale Wandthromben | Nicht beurteilbar |

Bei dem Alkylhydroxid handelt es sich insbesondere um Ethanol.

Rasterelektronenmikroskopische Begutachtung: Bei 50- bis 10.000 facher Vergrößerung zeigten sich ebenso unterschiedliche Eigenschaften, wie in Figs. 5 a bis d gezeigt, wobei Fig. 5 a) eine 10.000-fache Vergrößerung der Silanolatprothese mit angehefteten Erythrozyten zeigt, Fig. 5 b) einen ausgeprägten Thrombus mit Fibrinnetz an der Zinkoberfläche bei 2.000-facher Vergrößerung zeigt, Fig. 5 c) eine Darstellung in 3.000-facher Vergrößerung der PVP-Oberfläche zeigt, an der die Erythrozyten locker aufliegen, ohne mit ihr verhaftet zu sein, wobei die Längsstreifen Artefakte bei der Herstellung der Prothese darstellen; und Fig. 5d) eine 1.000-fache Vergrößerung einer Albumin-beschichteten Polyesterprothese nach dem Stand der Technik ist, an der Proteine und Erythrozyten anhaften.

Allgemeine Gerinnungsparameter: Es erfolgte eine weitere Analyse anhand von Routine-Gerinnungsparametern. Vor Heparin-Gabe hatten sich Normwerte gezeigt, nach 3000 iE Heparin i.v. war die PTT des Probanden erwartungsgemäß auf über 150 Sekunden angestiegen. Nach 60 Minuten Kontakt hatte sich diese Zeit für alle Prothesen reduziert. Die besten Werte wiesen Alkylhydroxid, PVA, PDMS, PVP und PC sowie die beschichtete Polyesterprothese auf Ein Thrombozyten- oder Erythrozytensturz war bei keinem Material aufgetreten. Zunächst wurden die Werte zu definieren Zeitpunkten nach Rotationsbeginn ermittelt (Kinetik). Hierbei zeigte sich für alle Materialien ein rascher Rückgang der PTT und der TZ unmittelbar nach Oberflächenkontakt, im weiteren Verlauf blieben diese Werte dann jedoch stabil. Der Quick-Wert wurde nicht beeinflusst.

ELISA-Analyse: Nach Bestimmung der allgemeinen Gerinnungsparameter wurden Silanolat, Zinksulfat und PEO von den weitergehenden Untersuchungen ausgeschlossen. In den ELISA-Untersuchungen zeigte sich eine unterschiedliche Aktivierung der Gerinnung und Fibrinolyse für die Prothesen.

Wir stellten fest, dass der reine Silikonschlauch (also die PDMS-Oberfläche) und die PC sowie die PVA-Beschichtung im Vergleich zu Polyesterprothesen einen günstigen Einfluß auf die Gerinnungsneigung hat. Andere Moleküle wiederum führten zu einer verstärkten Gerinnungsneigung. Diese bestätigte sich makroskopisch, elektronenmikroskopisch und in den Laborwerten (Tabelle 7)

Tabelle 7: ELISA-Analysen nach Oberflächenkontaktierung. PC und PDMS wiesen die günstigsten Werte auf (4 Versuchsgänge, jeweils 2 Proben nach 60 Minuten Oberflächenkontaktierung). Angegeben sind Mittelwert und Standardabweichung für Prothrombin F1&2 (Marker für Aktivität der Gerinnung), D-Dimere (Marker für Aktivität der Fibrinolyse) und β-Thromboglobulin (Aktivierung der Plättchen).

| Oberfläche/ Parameter | Leer | PVP | AH | PVA | PC | PDMS |
|---|---|---|---|---|---|---|
| Prothrombin F1&2 (nmol/l) | 0,88 | 0,80±0,08 | 0,96±0,23 | 0,95±0,28 | 0,84±0,28 | 0,83±0,22 |
| D-Dimere (ng/ml) | 286,5 | 299±43,1 | 301±7,1 | 278±19,1 | 214±36,8 | 249±49,5 |
| β-Thromboglobuli n (IU/ml) | 95 | 93,3±17,3 | 92,4±13,6 | 93,3±17,3 | 88,6±5,0 | 89,3±10,0 |

In der Kinetik ergaben sich keine einheitlichen Resultate. PVP und PC wiesen eine Erhöhung von Prothrombin F+2 unmittelbar nach Kontakt auf als Hinweis auf eine Aktivierung der Gerinnung, im weiteren Verlauf fielen diese Werte jedoch ab (Fig. 6 a). Alkylhydroxid, PVA und PDMS dagegen wiesen weder einen Anstieg, noch einen Abfall im Verlauf auf, wobei die Ergebnisse in Fig. 6 dargestellt sind in Form der ELISA-Analysen, angegeben 5, 15, 30, 60 und 120 Minuten nach Beginn der Rotation, wobei Fig. 6 a) Prothrombin F1 + F2, Fig. 6 b) D-Dimere, und Fig. 6 c) β-Thromboglobulin darstellt und die verschiedenen Oberflächen wie folgt bezeichnet sind:

PVP = Polyvinylpyrrolidon;
AH = Alkylhydroxid;
PVA = Polyvinylalkohol;
PC = Phosphocholin;
PDMS = Polydimethylsiloxan.

Für die D-Dimere und somit die Aktivierung der Fibrinolyse fand sich für Alkylhydroxid ein deutlicher Anstieg direkt nach Oberflächenkontakt (Fig. 6 b). β-Thromboglobulin und damit die Aktivierung der Plättchen erwies sich als erniedrigt für PVA (jedoch mit zunehmendem Spiegel im Laufe der Zeit), PDMS und insbesondere für PC (Fig. 6 c). Für Tissue factor und Factor XIIa (beide nicht dargestellt) konnte mit Ausnahme einzelner Ausreißer kein signifikante Tendenz festgestellt werden, für alle Prothesen fanden sich in nahezu allen Messungen Normwerte.

### Beispiel 5: Elution von Molekülen von der Prothese-Untersuchung im Kreislaufmodell

Die Elution von Molekülen wurde zunächst im Kreislaufmodell, und dann ex-vivo (Rinderarterie) nachgewiesen. Untersucht wurden folgende Substanzen: Acetylsalicylsäure (ASS), Sudanrot, Gentamycin, Sirolimus und Immunsuppressivum IS. Als Medium diente Humanalbumin 5% bei Raumtemperatur.

Um die Auswaschung von ASS zu testen, wurde der pH-Wert des Mediums in regelmäßigen Abständen ermittelt, und so eine semiquantitative Einschätzung erreicht. Sudanrot, ein hydrophober Farbstoff wurde photometrisch analysiert. Die Peeks für die Photometrie liegen bei 375 und 540 nm Wellenlänge. Sirolimus und IS wurden durch quantitative Spiegelbestimmung nachgewiesen (Labor Limbach, Heidelberg sowie Zentrallabor, Universitätsklinik Würzburg). Für die ex-vivo Analyse wurde die multifunktionelle Prothese mit einer Rinderarterie (4 mm, Procol®, Fa. LA Med) anastomosiert und im Kreislaufmodell implantiert. Nach 24 Stunden wurden dann Wandstücke der Arterie in definierten Abständen zur Anastomose herausgeschnitten, homogenisiert, und zentrifugiert. Im Überstand wurde der Sirolimus bzw. IS Spiegel gemessen.

Die ersten Prothesen enthielten ASS (Acetylsalicylsäure) in unterschiedlichen Konzentrationen. Anhand des pH-Wert-Abfalles im Medium um 2 Einheiten innerhalb einer Woche konnten wir den Nachweis der sukzessiven Elution dieses hydrophilen, sauren Moleküls nachweisen.

Um die Elution eines hydrophoben Moleküls zu untersuchen, wurde Sudanrot in die Prothese inkorporiert, wobei die Verteilung des Sudanrots im Bereich der Kontaktflächen in Fig. 7 gezeigt ist. In den regelmäßig entnommenen Proben konnte jedoch zu keinem Zeitpunkt dieser Farbstoff photometrisch in der Flüssigkeit nachgewiesen werden. Stattdessen nahmen die angrenzenden Schläuche die Farbe an, in Flussrichtung deutlich mehr als in der Gegenrichtung. So konnte die Abgabe des hydrophoben Stoffes an das umliegende Gewebe bewiesen werden.

Im nächsten Schritt wurden biologisch wirksame Moleküle in die Prothese eingebracht. Das Immunsuppressivum IS wurde sukzessive aus den Prothesen in das Medium abgegeben, wobei die Freisetzung des Immunsuppressivums IS im Medium in Fig. 8 a) und in der angrenzenden Rinderarterie in Fig. 8 b) dargestellt ist. Nach 30 Minuten war erstmals der Stoff im Medium nachweisbar, um nach 30 Stunden einen Maximalwert zu erreichen. In Bezug auf die Menge an zirkulierendem Medium konnten wir eine Abgabe von 2-3 $\mu$g IS pro Stunde errechnen. Im weiteren Verlauf kam es zu einer Abnahme der Konzentration.

Nach 1 Woche hatte sich der Spiegel im Medium bei 8-9 $\mu$g/l eingestellt. Nach Wechsel des Mediums und Entfernen des Bypasses wurde nach 2 Stunden ein Spiegel von 3 $\mu$g/l gemessen. Es ist also davon auszugehen, dass der Stoff in die Silikonschläuche des Kreislaufmodells inkorporiert wurde, und diesen dann im Sinne eines Gleichgewichtssystems wieder abgab.

Die Versuche wurden auch für Sirolimus vorgenommen. In der Prothese wurde eine Konzentration von 1,3 $\mu$g/cm$^2$ gemessen. Es kam rasch zu einem Anstieg im Medium auf 7 ng/ml, das Maximum lag bei 9 ng/ml.

Ex-Vivo-Versuch: Eine solche Prothese wurde mit einer Rinderarterie anastomosiert. Nach 24 Stunden wurde der IS-Gehalt im Gewebe der angrenzenden Rinderarterie bestimmt. Hierbei zeigte sich eine direkte Abhängigkeit von der Entfernung. Unmittelbar an der Anastomose lag der Wert > 30 $\mu$g/l/mm$^2$, 3 mm von der Anastomose bei 22,8 $\mu$g/l/mm$^2$, 5 mm entfernt bei 14,2 $\mu$g/l/mm$^2$, weiter entfernt wurden keine Werte mehr erreicht. Mit diesem Versuch konnte der Nachweis der Elution in organisches Gewebe erbracht werden.

**Beispiel 6: Elution von Molekülen von der Prothese-Untersuchung im Tiermodell**

Im letzten Schritt wurden die Prothesen in einem vorläufigen Tierversuch implantiert, um den Einfluss auf die Intimahyperplasie aufzuzeigen. Als Versuchstiere dienten 6 Göttinger Mini-Schweine. In i.v. Vollnarkose (Thiopental®) wurden die Aa. Iliacae über einen transperitonealen Zugang freigelegt, und in jedem Tier je eine multifunktionelle und eine herkömmliche Prothese eingesetzt. Alle Tiere erhielten eine Antibiotikaprophylaxe (Spizef®) und wurden mit Heparin antikoaguliert. Je zweimal wurden als Medikamente Paclitaxel, Sirolimus und IS verwendet. Wir haben Patch-Plastiken angelegt, da die Prothesen-Arterien-Grenzlinie größer ist als bei End-zu-End Anastomosen, und wir so die intimale Hyperplasie im Querschnitt gut beurteilen können. Die Tiere wurden nach 6 Wochen erneut operiert, und die Iliacagabeln explantiert. Von den Patch-Plastiken wurden im mittleren Bereich Quer- und im proximalen bzw. distalen Bereich Längsproben gewonnen. Diese wurden in Formaldehyd fixiert. Die weitere Bearbeitung umfasst die Dehydrierung in aufsteigender Alkoholreihe bis zur Einbettung in Paraffin. Die Präparate wurden geschnitten und mit HE und Tri-PAS gefärbt. Beurteilt wurden neben der Ausprägung der IH das Einwachs-Verhalten und entzündliche Reaktionen.

Alle Operationen konnte ohne Besonderheiten durchgeführt werden, in allen Fällen waren Patch-Plastiken im Bereich der Iliacalgabeln angelegt worden. Im weiteren Verlauf gab es keine Auffälligkeiten, alle Tiere erholten sich rasch von der Operation. Klinisch hatten sich bei keinem Tier Ischämiezeichen gezeigt.

Nach 6 Wochen wurden die Tiere getötet und die Iliaca-Gabeln explantiert. Alle Prothesen waren gut im Retroperitoneum eingeheilt. Es ergab sich kein Hinweis für eine verzögerte Wundheilung oder Infektion, wie in Fig. 9 dargestellt, wobei Fig. 9 a) die gute Einheilung der erfindungsgemäßen Protease an der explantierten Iliacagabel und Fig. 9 b) die Histologie davon zeigt.

Auch histologisch gab es keine Unterschiede im Einheilungsverhalten zwischen den herkömmlichen und den neuen, Silikon-beschichteten, Medikamenten-tragenden Prothesen. Es zeigten sich für alle Prothesen noch Fremdkörperreaktionen.

Für die Immunsuppressiv-beschichteten Materialien konnte eine Reduktion der Intimahyperplasie gezeigt werden. Dieser Effekt wurde sowohl für IS, als auch für Sirolimus beobachtet und ist in Fig. 10 dargestellt. Fig. 10 a) und Fig. 10 b) zeigen dabei herkömmliche Prothesen, an denen man den Randwall zwischen Patch und Arterie (a) erkennt, was einer Intimahyperplasie (b) entspricht. Fig. 10 c) und d) zeigen die Prothese gemäß der vorliegenden Erfindung, wobei hier kein Randwall auftritt und in der in Fig. 10 d) gezeigten histologischen Darstellung nur eine geringe Ausprägung der Intimahyperplasie beobachtet wurde.

Bei Verwendung von Paclitaxel wurde eine komplette Unterdrückung der Endothelialisierung beobachtet, wobei der Patch von außen gut eingeheilt war, wie aus Fig. 11 ersichtlich ist.

Die in der vorangehenden Beschreibung, den Ansprüchen und den Zeichnungen offenbarten Merkmale der Erfindung können sowohl einzeln als auch in beliebiger Kombination zur Verwirklichung der Erfindung in ihren verschiedenen Ausführungsformen wesentlich sein.

**Patentansprüche**

1. Prothese umfassend ein proximales und ein distales Ende mit einem sich dazwischen erstreckenden Hohlraum, wobei die Prothese eine Trägerstruktur und mindestens eine biologisch aktive Verbindung umfasst, die Trägerstruktur aus einem Trägermaterial und einem Beschichtungsmaterial besteht, wobei das Beschichtungsmaterial Polydimethylsiloxan ist, wobei die Beschichtung der Prothese 10 - 30 mg Polydimethylsiloxan/cm$^2$ Oberfläche beträgt.

2. Prothese nach Anspruch 1, **dadurch gekennzeichnet, dass** die Beschichtung der Prothese 15 - 26 mg Polydime-thylsiloxan/cm$^2$ Oberfläche beträgt.

3. Prothese nach Anspruch 2, **dadurch gekennzeichnet, dass** die Beschichtung der Prothese 16 - 23 mg Polydime-thylsiloxan/cm$^2$ Oberfläche beträgt.

4. Prothese nach einem der Ansprüche 1 bis 3, **dadurch gekennzeichnet, dass** das Trägermaterial ausgewählt ist aus der Gruppe, die Polytetrafluoroethylen, Polyester und Polyurethan umfasst.

5. Prothese nach einem der Ansprüche 1 bis 4, **dadurch gekennzeichnet, dass** das Trägermaterial und das Beschichtungsmaterial eine Matrix ausbilden.

6. Prothese nach Anspruch 5, **dadurch gekennzeichnet, dass** die Matrix die biologisch aktive Verbindung enthält.

7. Prothese nach einem der Ansprüche 1 bis 6, **dadurch gekennzeichnet, dass** die Trägerstruktur eine Außenwand und eine Innenwand definiert, wobei die Innenwand den Blut führenden Hohlraum und die Außenwand die Trägerstruktur gegen das umgebende Gewebe begrenzt.

8.   Prothese nach Anspruch 7, **dadurch gekennzeichnet, dass** die Prothese flüssigkeitsdicht ist.

9.   Prothese nach einem der Ansprüche 1 bis 8, **dadurch gekennzeichnet, dass** das Beschichtungsmaterial eine Modifikation aufweist.

10.   Prothese nach Anspruch 9, **dadurch gekennzeichnet, dass** die Modifikation aus der Gruppe ausgewählt ist, die Silanolat, Alkylhydroxid, Polyvinylalkohol, Polyethylenoxid, Zinksulfat, Polyvinylpyrrolidon und Phosphocholin umfasst.

11.   Prothese nach Anspruch 10, **dadurch gekennzeichnet, dass** die Modifikation aus der Gruppe ausgewählt ist, die Polyvinylalkohol, Polyvinylpyrrolidon und Phosphocholin umfasst.

12.   Prothese nach einem der Ansprüche 9 bis 11, **dadurch gekennzeichnet, dass** die Modifizierung an der Innenwand vorhanden ist und bevorzugterweise dort dauerhaft verbleibt.

13.   Prothese nach einem der Ansprüche 1 bis 12, **dadurch gekennzeichnet, dass** mindestens eine biologisch aktive Verbindung in der Matrix vorhanden ist.

14.   Prothese nach einem der Ansprüche 1 bis 13, **dadurch gekennzeichnet, dass** die biologisch aktive Verbindung aus der Gruppe ausgewählt ist, die Antibiotika, Immunsuppressiva und Proliferationshemmer umfasst.

15.   Prothese nach einem der Ansprüche 1 bis 14, **dadurch gekennzeichnet, dass** die biologisch aktive Verbindung aus der Gruppe ausgewählt ist, die Acetylsalicylsäure, Sirolimus und Paclitaxel umfasst.

16.   Prothese nach einem der Ansprüche 1 bis 15, insbesondere nach einem der Ansprüche 8 bis 15, **dadurch gekennzeichnet, dass** die biologisch aktive Verbindung mit einer Rate von 2 - 3 $\mu$g/Stunde abgegeben wird.

17.   Prothese nach einem der Ansprüche 1 bis 16, **dadurch gekennzeichnet, dass** die Prothese eine Gefäßprothese ist.

18.   Prothese nach einem der Ansprüche 1 bis 17, **dadurch gekennzeichnet, dass** die Prothese bei der kniegelenksübergreifenden alloplastischen Rekonstruktion verwendbar ist.

19.   Prothese nach einem der Ansprüche 1 bis 18, **dadurch gekennzeichnet, dass** die Prothese als pheripherer oder zentraler AV-Shunt verwendbar ist.

20.   Prothese nach einem der vorangegangenen Ansprüche **dadurch gekennzeichnet, dass** die Prothese als Patch oder als Dialyse-Shunt verwendbar ist.

**Claims**

1.   Prosthesis comprising a proximal and a distal end with a hollow space extending between them, which prosthesis comprises a carrier structure and at least one biologically active compound, which carrier structure consists of a carrier material and of a coating material, which coating material is polydimethylsiloxane, and the prosthesis coating has a 10 - 30 polydimethylsiloxane/cm$^2$ surface.

2.   The prosthesis according to Claim 1, **characterized in that** the coating of the prosthesis has a 15 - 26 mg poly-dimethylsiloxane/cm$^2$ surface.

3.   The prosthesis according to Claim 2, **characterized in that** the coating of the prosthesis has a 16 - 23 mg poly-dimethylsiloxane/cm$^2$ surface.

4.   The prosthesis according to one of Claims 1 to 3, **characterized in that** the carrier material is selected from the group comprising polytetrafluoroethylene, polyester and polyurethane.

5. The prosthesis according to one of Claims 1 to 4, **characterized in that** the carrier material and the coating material form a matrix.

6. The prosthesis according to Claim 5, **characterized in that** the matrix contains the biologically active compound.

7. The prosthesis according to one of Claims 1 to 6, **characterized in that** the carrier structure defines an outer wall and an inner wall, which inner wall delimits the hollow space guiding the blood and which outer wall delimits the carrier structure from the surrounding tissue.

8. The prosthesis according to Claim 7, **characterized in that** the prosthesis is liquid-tight.

9. The prosthesis according to one of Claims 1 to 8, **characterized in that** the coating material has a modification.

10. The prosthesis according to Claim 9, **characterized in that** the modification is selected from the group comprising silanolate, alkyl hydroxide, polyvinyl alcohol, polyethylene oxide, zinc sulphate, polyvinylpyrrolidone and phosphocholine.

11. The prosthesis according to Claim 10, **characterized in that** the modification is selected from the group comprising polyvinyl alcohol, polyvinylpyrrolidone and phosphocholine.

12. The prosthesis according to one of Claims 9 to 11, **characterized in that** the modification is present on the inner wall and preferably remains there permanently.

13. The prosthesis according to one of Claims 1 to 12, **characterized in that** at least one biologically active compound is present in the matrix.

14. The prosthesis according to one of Claims 1 to 13, **characterized in that** the biologically active compound is selected from the group comprising antibiotics, immunosuppressive agents and proliferation inhibitors.

15. The prosthesis according to one of Claims 1 to 14, **characterized in that** the biologically active compound is selected from the group comprising acetylsalicylic acid, sirolimus and paclitaxel.

16. The prosthesis according to one of Claims 1 to 15, **characterized in that** the biologically active compound is released at a rate of 2 - 3 $\mu$g/hour.

17. The prosthesis according to one of Claims 1 to 16, **characterized in that** the prosthesis is a vascular prosthesis.

18. The prosthesis according to one of Claims 1 to 17, **characterized in that** the prosthesis can be used in alloplastic reconstruction extending over the knee joint.

19. The prosthesis according to one of Claims 1 to 18, **characterized in that** the prosthesis can be used as a peripheral or central AV shunt.

20. The process is according to one of the previous claims, **characterized in that** the prosthesis can be used as a patch or as a dialysis shunt.


**Revendications**

1. Prothèse présentant une extrémité proximale et une extrémité distale avec une cavité qui s'étend entre elles, la prothèse comprenant une structure porteuse et au moins un composé biologiquement actif, où la structure porteuse se compose d'un matériau substrat et d'un matériau de revêtement, le matériau de revêtement est du polydiméthylsiloxane et le revêtement de la prothèse contient de 10 à 30 mg de polydiméthylsiloxane par cm$^2$ de superficie.

2. Prothèse selon la revendication 1, **caractérisée en ce que** le revêtement de la prothèse contient de 15 à 26 mg de polydiméthylsiloxane par cm$^2$ de superficie.

3. Prothèse selon la revendication 2, **caractérisée en ce que** le revêtement de la prothèse contient de 16 à 23 mg

de polydiméthylsiloxane par cm$^2$ de superficie.

**4.** Prothèse selon l'une des revendications 1 à 3, **caractérisée en ce que** le matériau substrat est choisi dans le groupe comprenant le polytétrafluoroéthylène, le polyester et le polyuréthane.

**5.** Prothèse selon l'une des revendications 1 à 4, **caractérisée en ce que** le matériau substrat et le matériau de revêtement forment une matrice.

**6.** Prothèse selon la revendication 5, **caractérisée en ce que** la matrice contient le composé biologiquement actif.

**7.** Prothèse selon l'une des revendications 1 à 6, **caractérisée en ce que** la structure porteuse définit une paroi externe et une paroi interne, la paroi interne délimitant la cavité de circulation du sang et la paroi externe délimitant la structure porteuse par rapport au tissu environnant.

**8.** Prothèse selon la revendication 7, **caractérisée en ce que** la prothèse est étanche aux liquides.

**9.** Prothèse selon l'une des revendications 1 à 8, **caractérisée en ce que** le matériau de revêtement présente une modification.

**10.** Prothèse selon la revendication 9, **caractérisée en ce que** la modification est choisie dans le groupe comprenant un silanolate, un hydroxyde d'alkyle, l'alcool polyvinylique, le polyoxyde d'éthylène, le sulfate de zinc, la polyvinylpyrrolidone et la phosphocholine.

**11.** Prothèse selon la revendication 10, **caractérisée en ce que** la modification est choisie dans le groupe comprenant l'alcool polyvinylique, la polyvinylpyrrolidone et la phosphocholine.

**12.** Prothèse selon l'une des revendications 9 à 11, **caractérisée en ce que** la modification est présente sur la paroi interne et y reste de préférence en permanence.

**13.** Prothèse selon l'une des revendications 1 à 12, **caractérisée en ce qu'**au moins un composé biologiquement actif est présent dans la matrice.

**14.** Prothèse selon l'une des revendications 1 à 13, **caractérisée en ce que** le composé biologiquement actif est choisi dans le groupe comprenant des antibiotiques, des immunosuppresseurs et des inhibiteurs de prolifération.

**15.** Prothèse selon l'une des revendications 1 à 14, **caractérisée en ce que** le composé biologiquement actif est choisi dans le groupe comprenant l'acide acétylsalicylique, le sirolimus et le paclitaxel.

**16.** Prothèse selon l'une des revendications 1 à 15, en particulier selon l'une des revendications 8 à 15, **caractérisée en ce que** le composé biologiquement actif est délivré au rythme de 2 à 3 μg par heure.

**17.** Prothèse selon l'une des revendications 1 à 16, **caractérisée en ce que** la prothèse est une prothèse vasculaire.

**18.** Prothèse selon l'une des revendications 1 à 17, **caractérisée en ce que** la prothèse peut être utilisée dans la reconstruction alloplastique de l'articulation du genou.

**19.** Prothèse selon l'une des revendications 1 à 18, **caractérisée en ce que** la prothèse peut être utilisée comme pontage artério-veineux périphérique ou central.

**20.** Prothèse selon l'une des revendications précédentes, **caractérisée en ce que** la prothèse peut être utilisée comme pièce vasculaire ou comme shunt de dialyse.

Fig.1

| Molekül | Strukturformel | Eigenschaften | Literatur |
|---|---|---|---|
| Silanolat | Silikon $CH_3$ / —Si—O⁻ / $CH_3$ | Negativ geladene Oberfläche, Radikalenbildung | Razzaboni B. L. et al., Environ.Health Perspect. 87: 337-341; Rogers S. D. et al., J.Chromatogr.A 892: 57-65 |
| Alkylhydroxid | Silikon H / —C—OH / H | Hydrophile und hydrophobe Anteile | Lim F. et al., Biomaterials 14: 537-545; Silver J. H. et al., Biomaterials 20: 1533-1543 |
| Polyvinylalkohol (PVA) | Silikon $\left( \begin{matrix} H & H \\ C & C \\ H & OH \end{matrix} \right)_n$ | Verminderte Thrombozytenadhärenz, hydrophil | Cholakis C. H. et al., Biomed. Mater. Res. 23: 399-415; Godo M. N. et al., Biomaterials 20: 1117-1126 |
| Polyethylenoxid (PEO) | Silikon $\left( \begin{matrix} H & H \\ C & C & O \\ H & H \end{matrix} \right)_n$ | Verringerte Proteinadhärenz | Gombotz W. R. et al., J. Biomed. Mater. Res. 25: 1547-1562; Ji J. et al., Biomaterials 22: 3015-3023 |

EP 1 663 337 B1

| Zinksulfat | | Negativ geladene Oberfläche | Petanova J. et al., Cent. Eur. J. Public Health 8: 137-140 |
|---|---|---|---|
| Silikon (Polydimethylsiloxan, PDMS) | | Biologisch inert, hydrophob, verminderte Thrombozytenadhärenz | Park J. H. et al., Biomaterials 23 : 1797-1808 |
| Polyvinylpyrrolidon (PVP) | | Hydrophil; Änderung der Proteinadhärenz | Boffa G. A. et al., J. Biomed. Mater. Res. 11: 317-337 |
| Phosphocholin (PC) | | Hydrophobe / lipophile Oberfläche; verringerte Thrombozytenadhärenz | Chen C. et al., Ann. Vasc. Surg. 11 : 74-79 ;  New G. et al., Catheter. Cardiovasc. Interv. 57: 266-271 |

Fig.2

23

Fig.3

Fig. 4

Fig. 5

26

Fig. 6

Fig. 7

Fig. 8

Fig. 9

Fig. 10

Fig. 11

EP 1 663 337 B1

IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- **BAUTERS C. et al.** *Cardiovasc.Res.,* vol. 31, 835-846
- **BEN SLIMANE S. et al.** *Eur.Surg.Res.,* vol. 20, 12-17
- **SONI A. B. et al.** *Int.J.Radiat.Oncol.Biol.Phys.,* vol. 54, 1174-1179
- **CONTE M. S. et al.** *J.Vasc.Surg.,* vol. 36, 1040-1052
- **TEEBKEN O. E. et al.** *Eur.J.Vasc.Endovasc.Surg.,* vol. 23, 475-485
- **ARTS C. H. et al.** *Eur.J.Vasc.Endovasc.Surg.,* vol. 23, 29-38
- **CONTE M. S. et al.** *J.Vasc.Surg.,* vol. 21, 413-421
- **GOMES D. et al.** *J.Vasc.Surg.,* vol. 34, 707-715
- **SEIFALIAN A. M. et al.** *Artif.Organs,* vol. 26, 307-320
- **IMIG, H ; GRUNDMANN, RT.** Gefäßprothesen - Wo geht es hin?. *Zentralbl. Chir.,* 2000, vol. 125, 298
- **EUGENE ROCHOW.** Silcon and Silicones. Springer Verlag
- **CARTER, AL et al.** *Cardiovascular Res.,* 2004, vol. 63, 617-624